# EUROPEAN PATENT APPLICATION

(11) **EP 1 366 773 A1**
(43) Date of publication of application: **03.12.2003**
(21) Application number: 02712308.2
(22) Date of filing: 13.02.2002
(51) Int. Cl.: A61K 45/00, A61K 38/17, A61K 31/711, A61K 48/00, A61P 43/00, A61P 35/00, A61P 29/00, G01N 33/15, G01N 33/50

(54) **CELL PROLIFERATION INHIBITORS COMPRISING ETS TRANSCRIPTION FACTOR OR GENE ENCODING THE SAME**

(30) Priority: 13.02.2001 JP 2001034834
(71) Applicant: HISAMITSU PHARMACEUTICAL CO. INC., Tosu-shi, Saga-ken 841-0017 (JP)
(72) Inventor: KAI, Hirofumi, Kumamoto-shi, Kumamoto 862-0949 (JP); HISATSUNE, Akinori, c/o HISAMITSU PHARMA. CO. INC., Tsukuba-shi, Ibaraki 305-0856 (JP)
(74) Representative: Cresswell, Thomas Anthony
(86) International application number: JP0201180
(87) International publication number: WO02064165

(57) **Abstract**

It is found out that an ETS transcription factor (more specifically, an ETS transcription factor MEF) has a potent effect of inhibiting cell proliferation and an effect of inhibiting MMP production. Based on this finding, novel cell proliferation inhibitors (more specifically, novel remedies for tumor and novel antirheumatics) with the use of the ETS transcription factor MEF or a gene encoding the same are provided. Namely, cell proliferation inhibitors comprising an ETS transcription factor or gene encoding the same or a substance controlling the effect of the ETS transcription factor or the gene encoding the same. Also, matrix metalloprotease (MMP) (more specifically, MMP-9) production inhibitors or IL-8 production inhibitors comprising the ETS transcription factor or gene encoding the same or a substance controlling the effect of the ETS transcription factor or the gene encoding the same are provided.

## Description

### Technical field

The present inventors have found that an ETS transcription factor having a gene transcription regulatory activity or a gene encoding the same, or a substance regulating a function of the ETS transcription factor or the gene encoding the same, more specifically, a transcription regulatory protein Myeloid Elf-1 like Factor (hereinafter referred to as MEF or MEF protein) or a gene encoding the same, or a substance regulating a function of the MEF protein or the gene encoding the same has functions of inhibiting cell proliferation such as a function of inhibiting cancer cell proliferation and a function of inhibiting synovial membrane fibroblast proliferation. Accordingly, the invention relates to a cell proliferation inhibitor comprising an ETS transcription factor or a gene encoding the same, or a substance regulating a function of the ETS transcription factor or the gene encoding the same, more specifically, an ETS transcription factor MEF protein or a gene encoding the same. Further, the invention relates to a matrix metalloprotease (MMP) production inhibitor, more specifically, an MMP-9 production inhibitor and an IL-8 production inhibitor, comprising an ETS transcription factor or a gene encoding the same, or a substance regulating a function thereof, more specifically, an ETS transcription factor MEF protein or a gene encoding the same, or a substance regulating a function thereof. Still further, the invention relates to a cell proliferation inhibitor, an antitumor agent or an antirheumatic agent based on these functions, and a method for screening a substance capable of being an active ingredient thereof.

### Background Art

Cells have a mechanism that after regular proliferation, they stop the proliferation. However, cancer cells performs endless proliferation by deviating from regular proliferation to invade normal tissues and destroy functions of normal tissues. Accordingly, in therapy of cancers, it is required that regularity is restored in the deviated endless proliferation of cancer cells, or proliferation of cancer cells is stopped, that is, they are destroyed by inhibiting invasion into normal tissues.

Antitumor agents typified by cisplatin and 5-fluorouracil and antitumor therapeutic methods such as radiotherapy and surgical excision have been to date developed. However, the problems in effects and side effects thereof have been pointed out. In the former, because of a less qualitative difference exerted on tumor cells and normal cells, the higher the antitumor effect, the more serious the side effect. Further, since sensitivity to agents varies with types of cancers, much care has to be taken in using antitumor agents. The latter therapeutic methods are quite effective because they excise or destroy selectively tumor cells temporally. However, since therapy marking invisible tumor cells is impossible, it always involves a risk of recurrence. Accordingly, instead of the foregoing therapeutic methods or therapeutic agents, attempts of antitumor therapy using genes encoding cytokines, cell period regulatory factors, transcription factors and the like have been made.

By the way, transcription factors regulate not only gene expression in cells but also functions of cells including differentiation and proliferation. Accordingly, transcription factors regulating functions of cells, namely, functions of cancer cells such as endless proliferation, invasion in normal cells and destruction of normal tissues can be used in gene therapy. With respect to an ETS (E26 transforming specific) transcription factor group, approximately 50 species ranging from drosophilae to humans have been reported. It has been known that the ETS transcription group has a DNA binding region comprising 85 amino acids having a high homology, recognizes a base sequence to which the ETS transcription group binds (hereinafter referred to as an ETS binding site) in common, and regulates expression of a gene that is specifically expressed in hemocytes. Further, it has been reported that the ETS transcription factor group not only has a gene expression regulatory activity but also participates in differentiation, proliferation and functional expression of many cells including blood cells. In regard to cancer cells, the ETS transcription factors might participate in proliferation or inhibition of cancer cells.

It has been reported that the ETS transcription factor PU.1 which is expressed only in monocytes, macrophages or lymphocytes is overexpressed in leukemia cells in differentiation by DMSO stimulation to induce cell death by apoptosis (Kihara-Negishi et al., Int. J. Cancer, 76(4), 523-530, 1998). Moreover, inhibition of endless proliferation of cancer cells is enabled by suppressing productive secretion of matrix metalloprotease (hereinafter referred to as MMP) which has been reported to participate in invasive metastasis and angiogenesis of cancer cells, or its activity. It has been reported that the ETS transcription factor PU.1 dramatically suppresses the promotor activity of MMP-1 in comparison to other transcription factors (Kahari et al., Oncogene, 14(22), 2651-2660, 1997).

### Disclosure of the Invention

The present invention has revealed that an ETS transcription factor, more specifically an ETS transcription factor MEF has a potent inhibitory function of cell proliferation, MMP production and IL-8 production. It provides a novel cell proliferation inhibitor, more specifically, a novel antitumor agent and a novel antirheumatic agent, using an ETS transcription factor MEF or a gene encoding the same.

### Brief Description of the Drawings

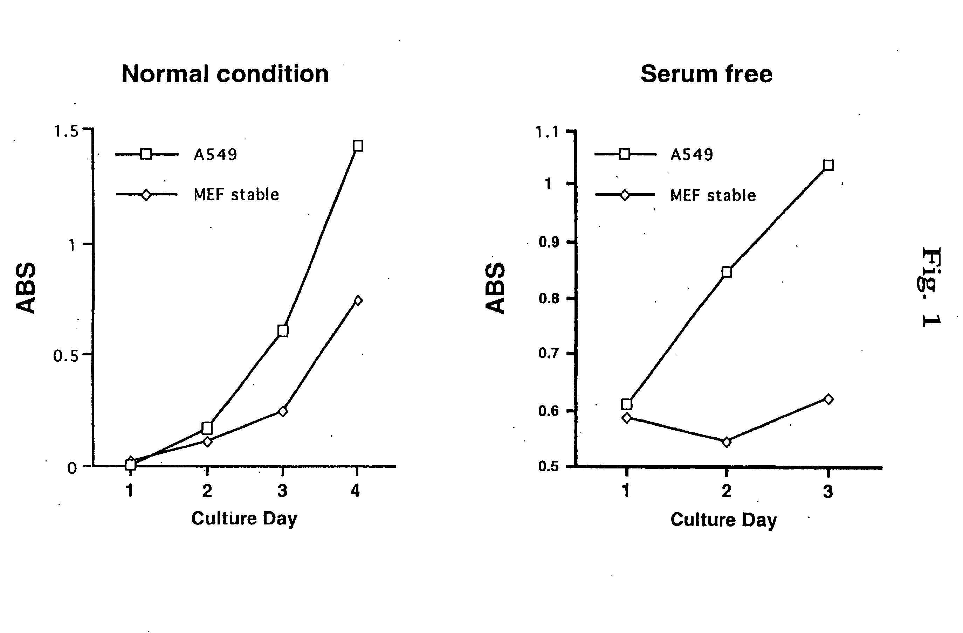

Fig. 1 is graphs illustrating cell proliferation activities of human pulmonary epithelial cell strain A549 and MEF gene stable overexpressing cell strain (No. 71) in the presence or absence of serum as measured by the MTT method.

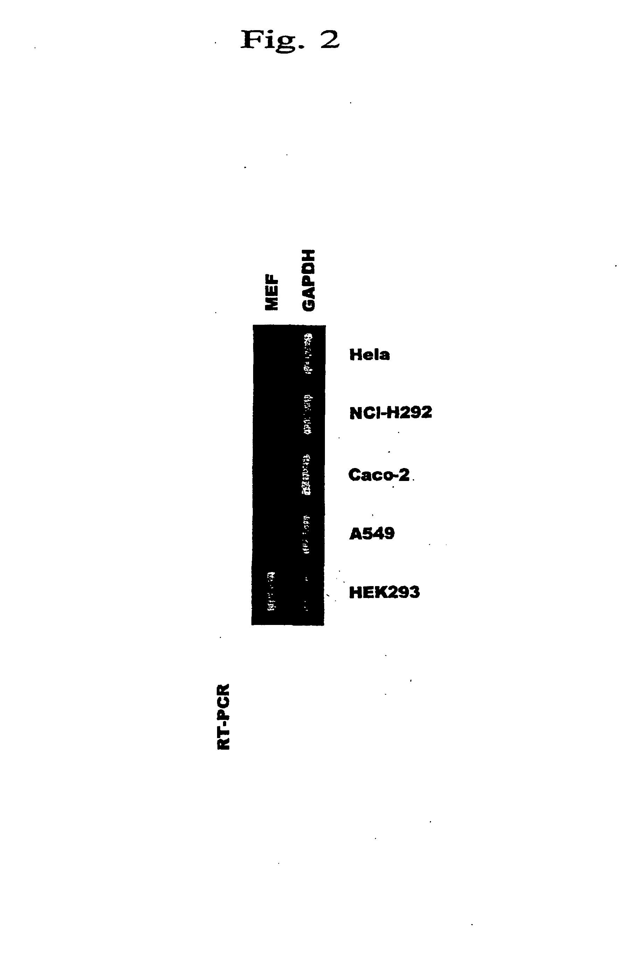

Fig. 2 is a photograph in place of a drawing, showing results of analyzing MEF expression in human cancer cells and normal cells by the RT-PCR method. GAPDH in Fig. 2 is shown as a control. In Fig. 2, HEK293 cell obtained from a human normal renal cell, A549 cell, Caco-2 cell, NCIH292 cell and Hela cell are shown from the left side.

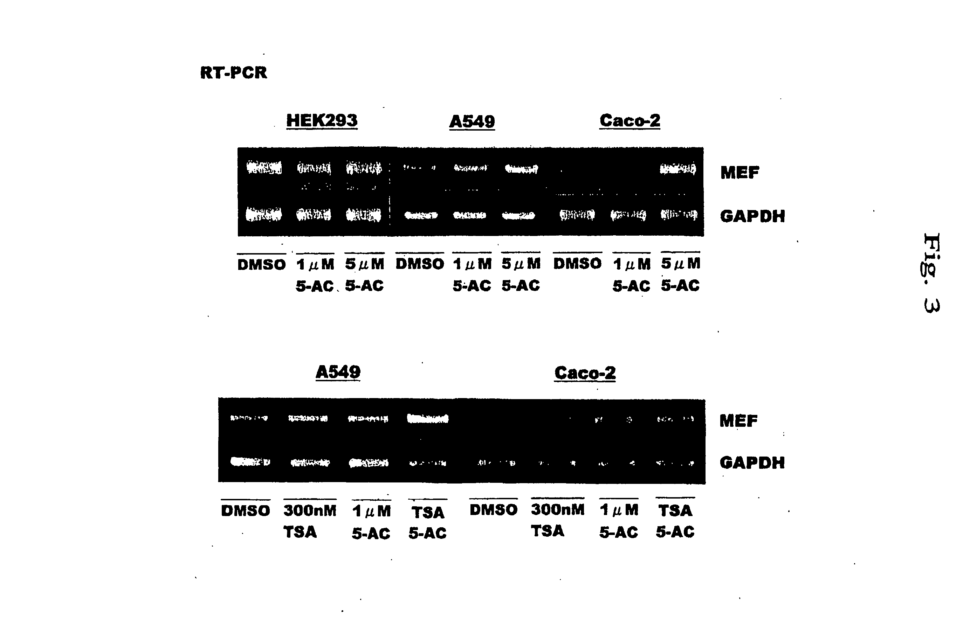

Fig. 3 is photographs in place of drawings, showing results of examining an effect given by a combined use of 5-aza-2'-deoxycytidine (5-AC) as a demethylation agent and tricostatin A (TSA) as a histone deacetylase inhibitor in MEF expression in human cancer cells and normal cells as measured by the RT-PCR method. GAPDH is shown as a control. In Fig. 3, HEK293 cell, *A549* cell and Caco-2 cell are shown from the left side of an upper panel, and A549 cell and Caco-2 cell from the left side of a lower panel. DMSO in Fig. 3 indicates dimethyl sulfoxide.

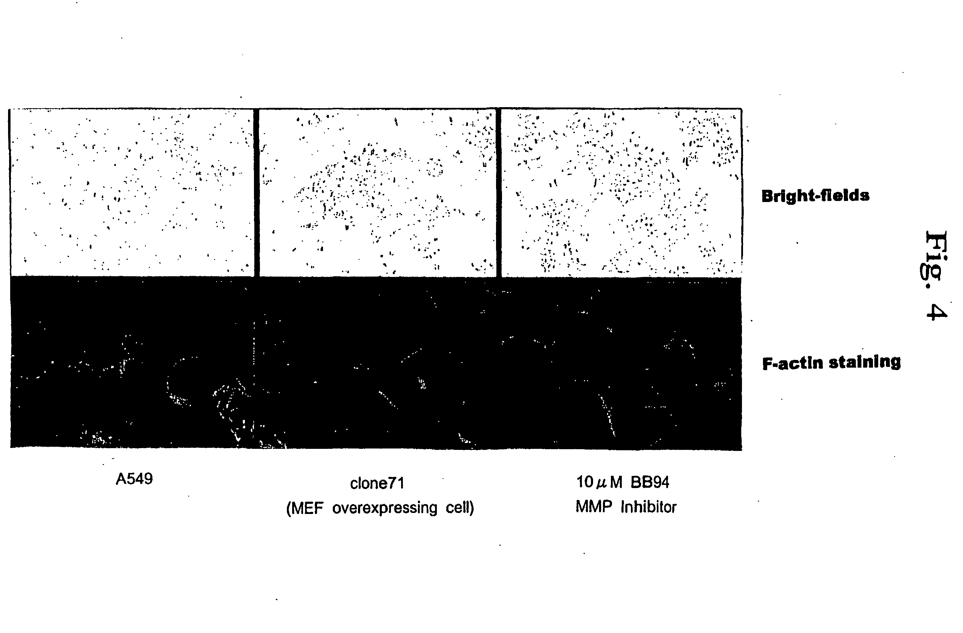

Fig. 4 is photographs in place of drawings, showing extracellular characteristics of MEF gene overexpressing cell line (clone 71). In Fig. 4, an upper panel shows a morphological condition, and a lower panel shows F-actin staining. In Fig. 4, A549 cell, MEF gene overexpressing A594 cell and A549 cell treated with BB94 (10 µM) are shown respectively from the left side.

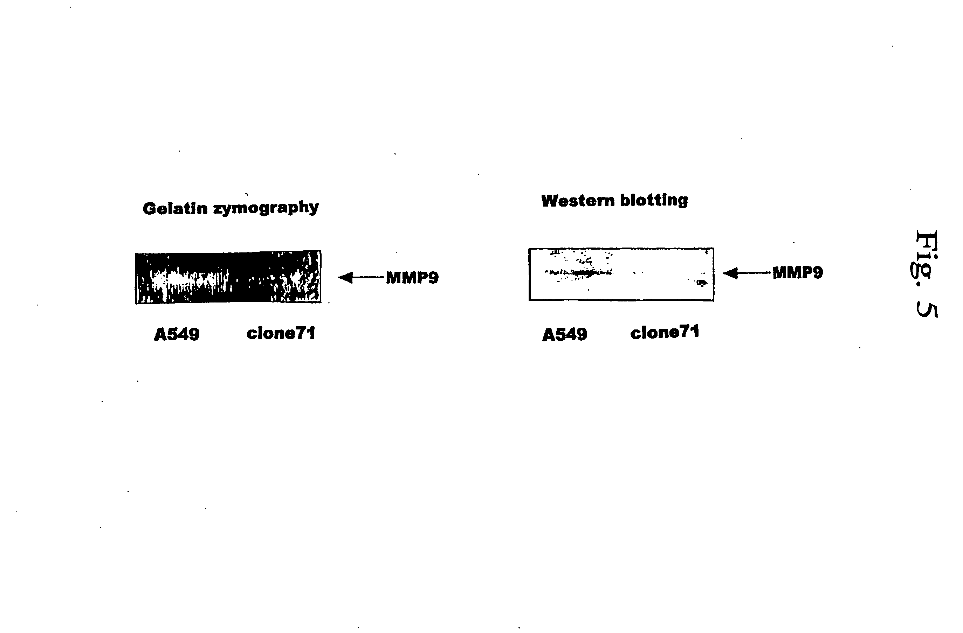

Fig. 5 is photographs in place of drawings, showing respectively results (right side in Fig. 5) of analyzing an MMP-9 protein content of A549 cell and MEF gene overexpressing cell in a serum-free culture supernatant by western blotting using anti-MMP-9 antibody, and results (left side in Fig. 5) of gelatin-zymography of MMP-9.

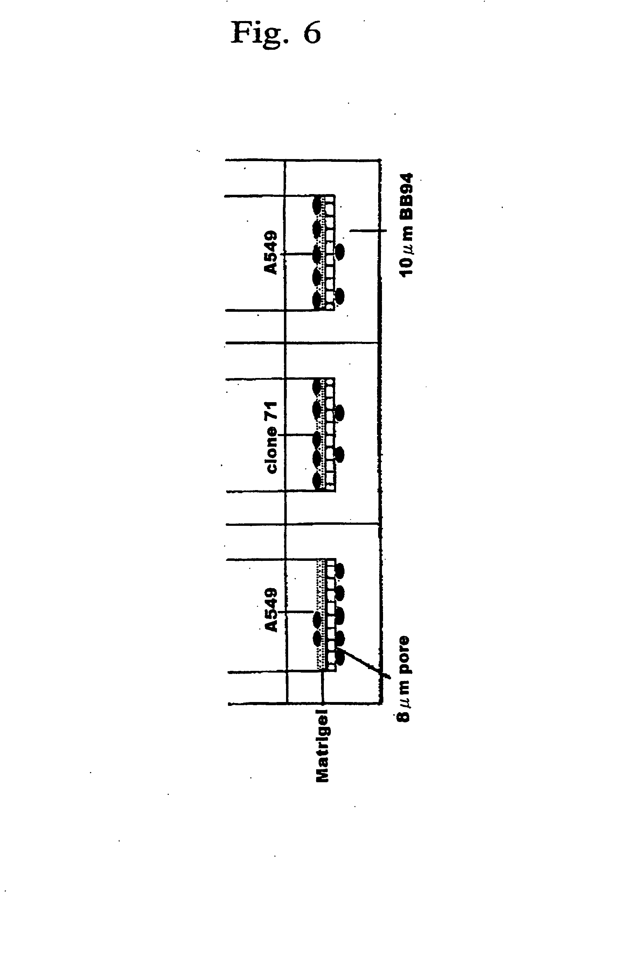

Fig. 6 is a diagrammatic view of an experiment of matrigel invasion assay for examining an invasion activity of A549 cells and MEF gene overexpressing cells. A549 cells (left side in Fig. 6), MEF gene overexpressing cells (center in Fig. 6) and A549 cells treated with BB94 (right side in Fig. 6) were inoculated respectively in an upper chamber in Fig. 6, and the numbers of cells on the lower side which were passed through a 8-µm filter were counted after 24 hours.

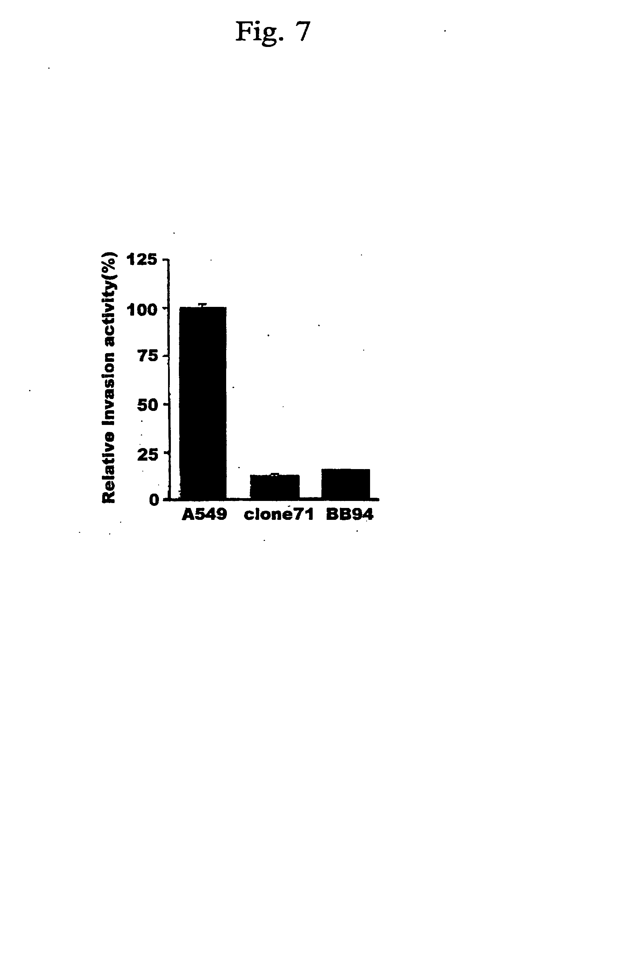

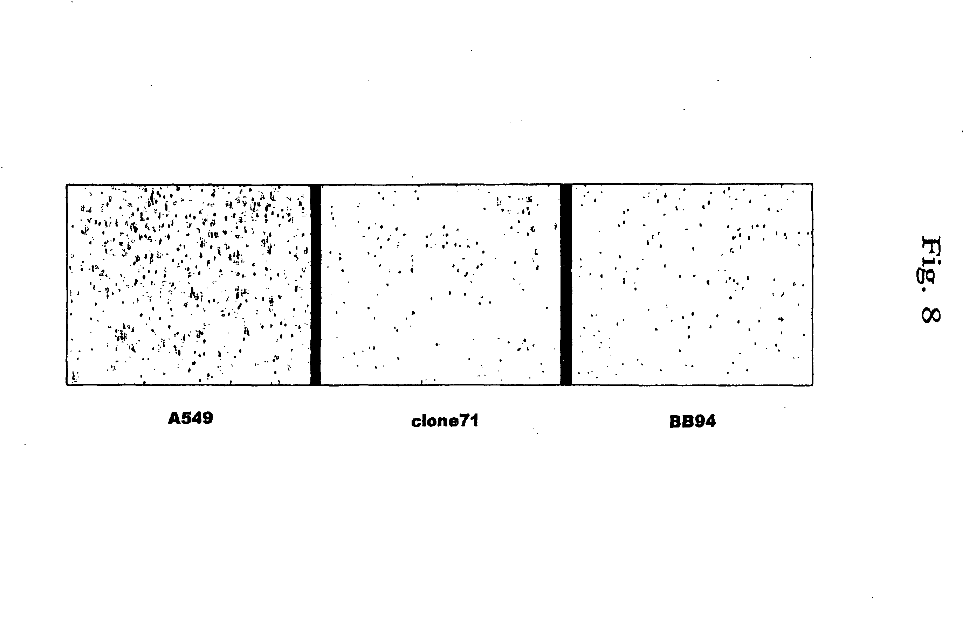

Fig. 7 is a graph showing the numbers of cells as a result of matrigel invasion assay shown in Fig. 6 in terms of percentage (%) relative to a control (A549 cell). Fig. 8 is a photograph in place of a drawing, showing conditions of the cells as a result of the matrigel invasion assay shown in Fig. 6. In Fig. 8, A549 cell (left side), MEF gene overexpressing cell (center) and A549 cell treated with BB94 (right side) are shown from the left side respectively.

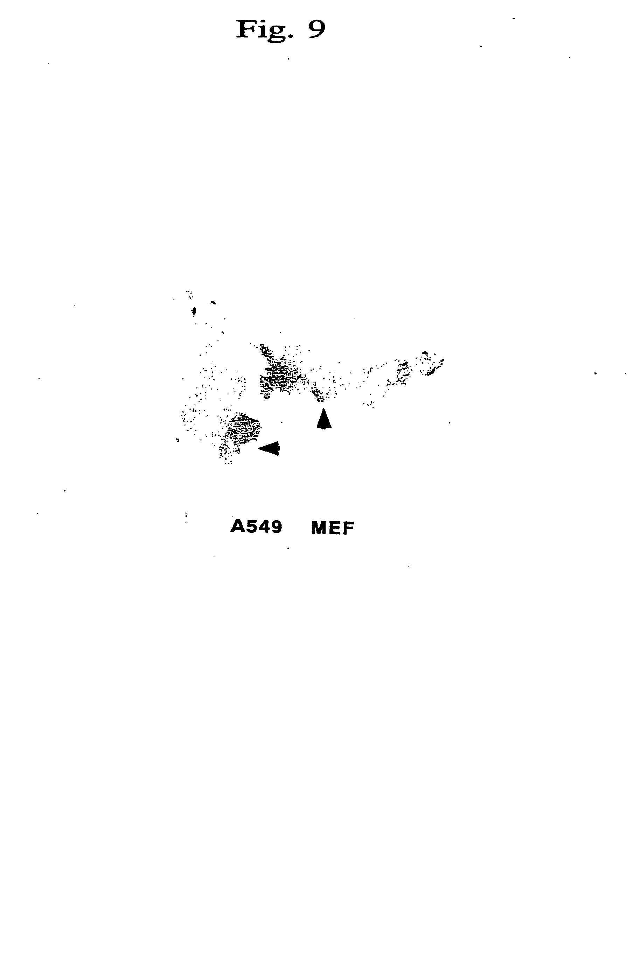

Fig. 9 is photographs in place of drawings, showing tumor formation after 2 months from subcutaneous inoculation of human pulmonary epithelial cell strain A549 and MEF gene stable overexpressing cell strain (No. 71) in the backs of nude mice.

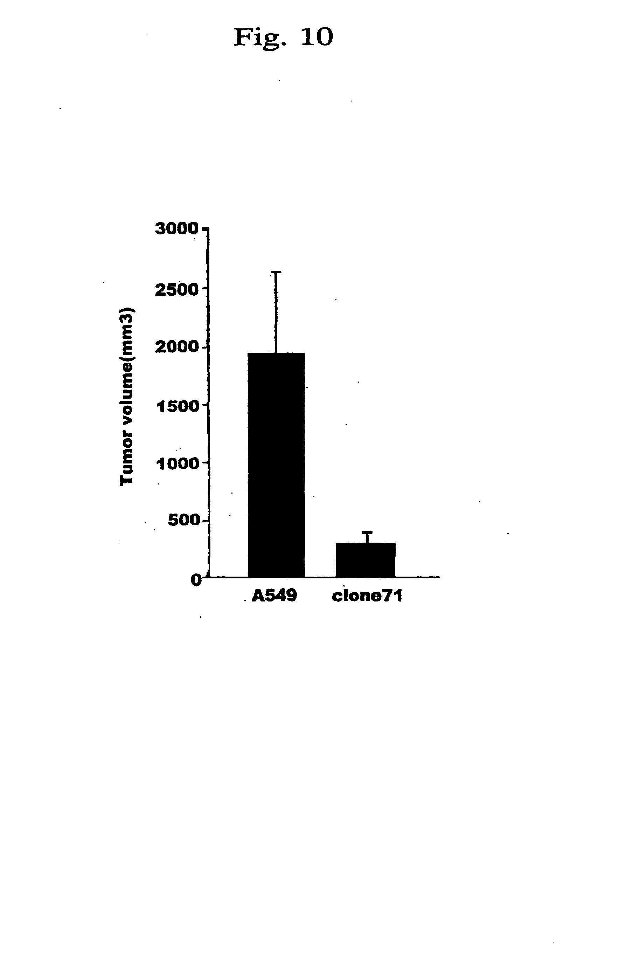

Fig. 10 is a graphical representation of a size of each tumor in nude mice in Fig. 9. In Fig. 10, the ordinate represents a volume (mm²) of a tumor. The left side shows A549 cell, and the right side shows MEF gene overexpressing cell.

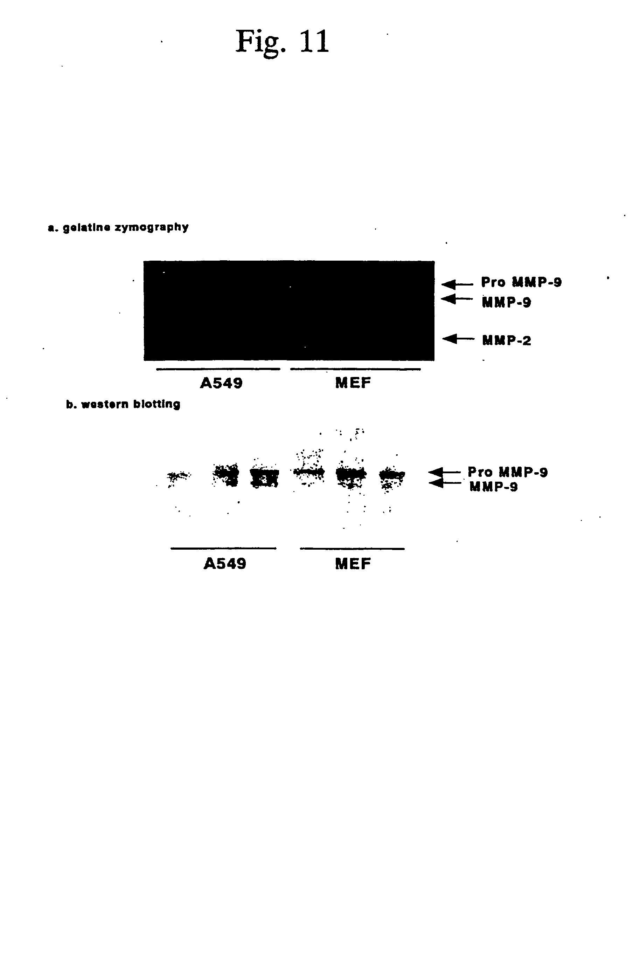

Fig. 11 is photographs in place of drawings, showing inhibition of MMP-9 production and activity in tumor tissues formed in nude mice with MEF gene stable overexpressing cell strain (No. 71) inoculated as measured by gelatin zymography and western blotting.

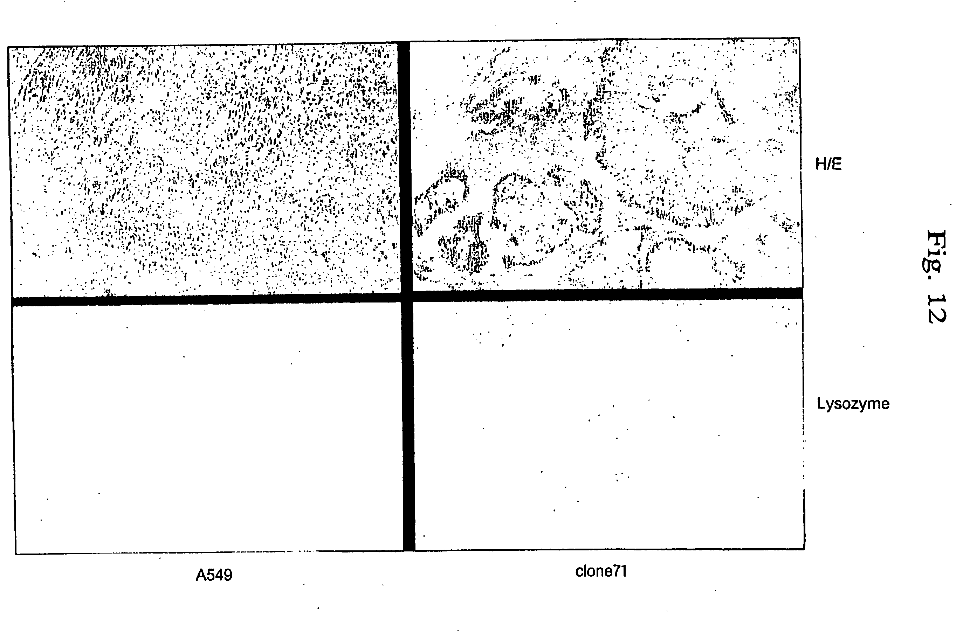

Fig. 12 is photographs in place of drawings, showing results of HE staining (upper panel in Fig. 12) of tumor cells in nude mice and results of lysozyme thereof (lower panel in Fig. 12).

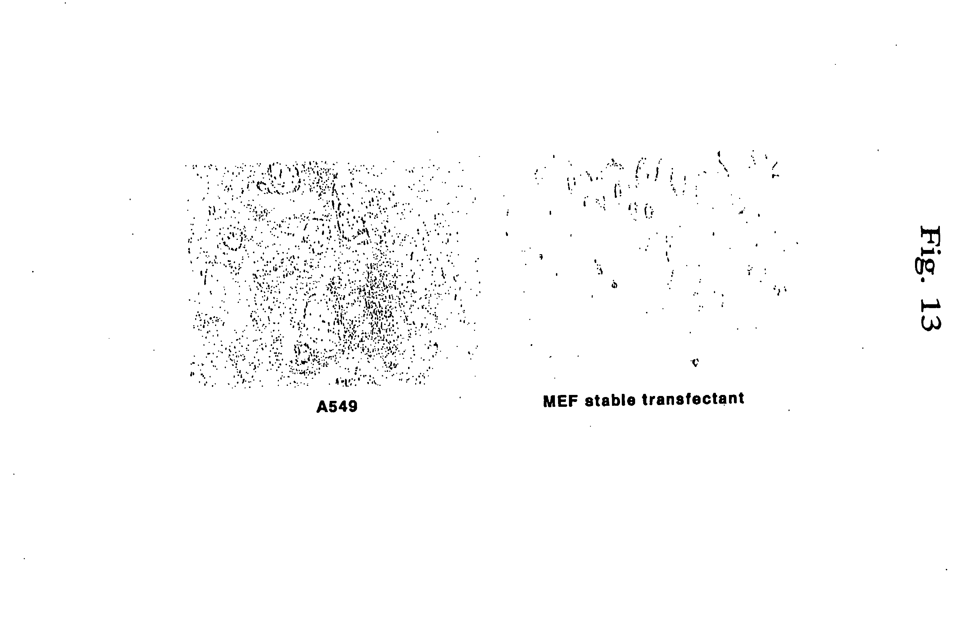

Fig. 13 is photographs in place of drawings, showing morphological changes of tumor tissues formed in nude mice with A549 and MEF gene stable overexpressing cell strain (No. 71) inoculated as measured by HE staining.

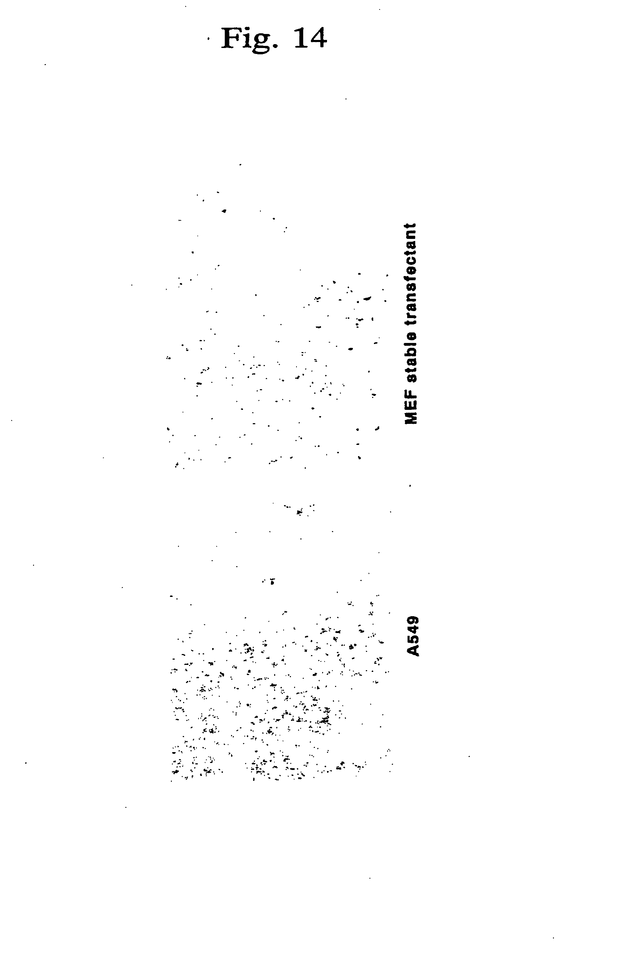

Fig. 14 is photographs in place of drawings, showing induction of apoptosis in tumor tissues formed in nude mice with MEF gene stable overexpressing cell strain (No. 71) inoculated as measured by the TUNEL method.

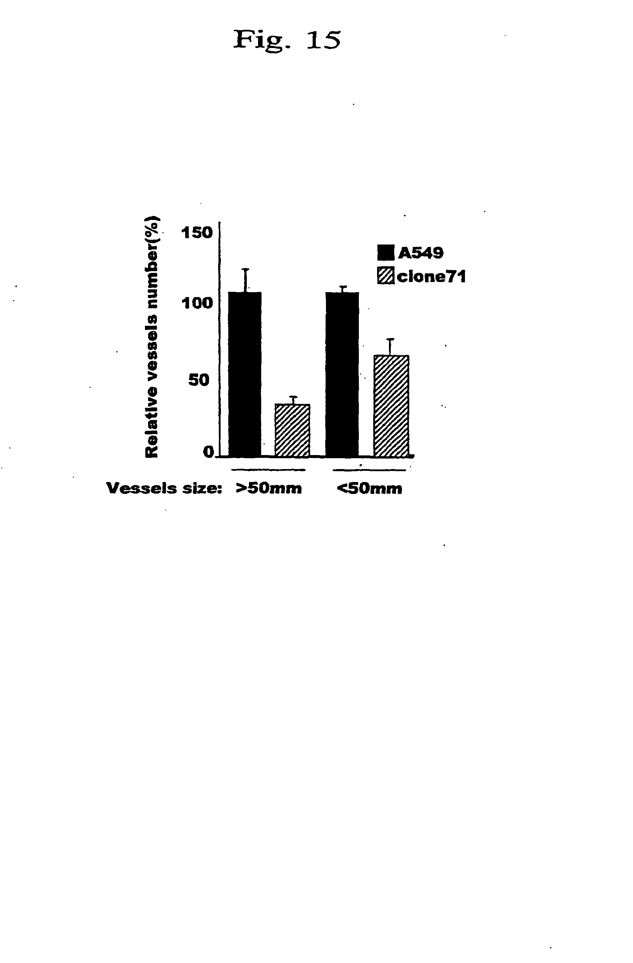

Fig. 15 is a graph showing results of analyzing angiogenesis of nude mice by immunological staining with CD31. In Fig. 15, the ordinate represents a value (%) relative to the vessels number of A549 cell, and the abscissa represents the vessels sizes of 50 mm or more and 50 mm or less. In these sizes, the left side shows the size of A549 cell, and the right side shows the size of MEF gene overexpressing cell (clone 71).

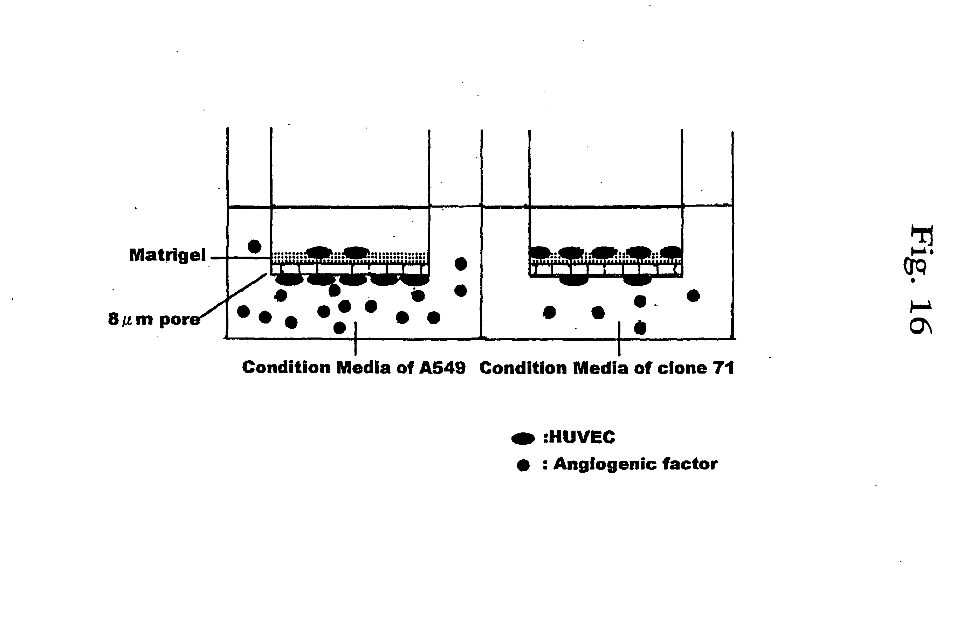

Fig. 16 is a diagrammatic view of an experiment on an influence of MEF gene overexpression of A549 cells on migration of vessel endothelial cells. In Fig. 16, a small black circle shows an angiogenetic factor, and an elliptic black circle shows a human umbilical vein endothelial cell (HUVEC). Human umbilical vein endothelial cells (HUVECs) were inoculated in an upper chamber, and cells migrating in a medium of a lower chamber were observed through a 8-µm filter.

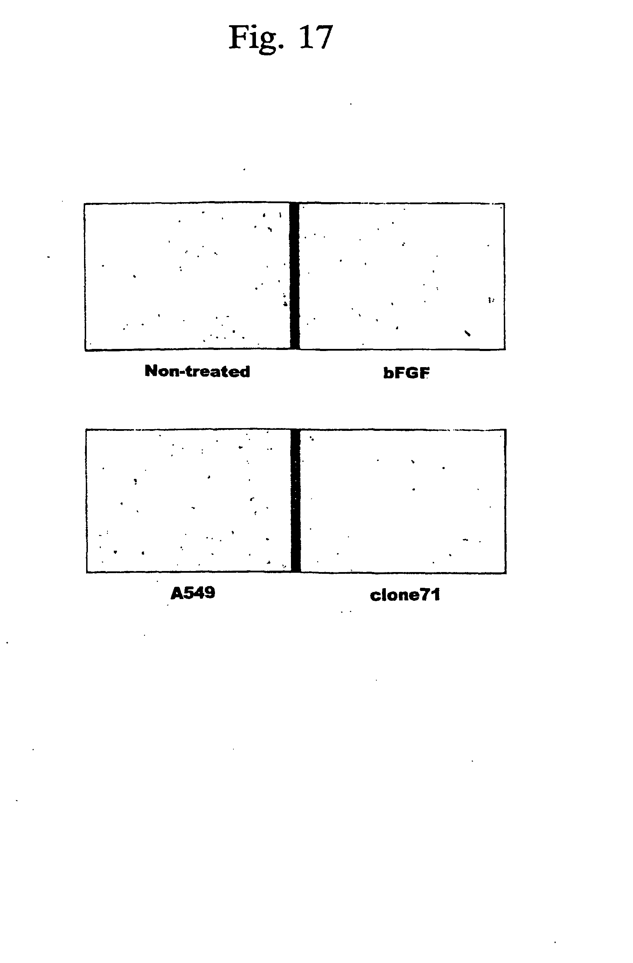

Fig. 17 is photographs in place of drawings, showing results of the experiment shown in Fig. 16. A non-treated case is shown in the left upper photograph, a case of adding bFGF in the right upper photograph, a case of a medium of A549 cell in the left lower photograph, and MEF gene overexpressing cell (clone 71) in the right lower photograph.

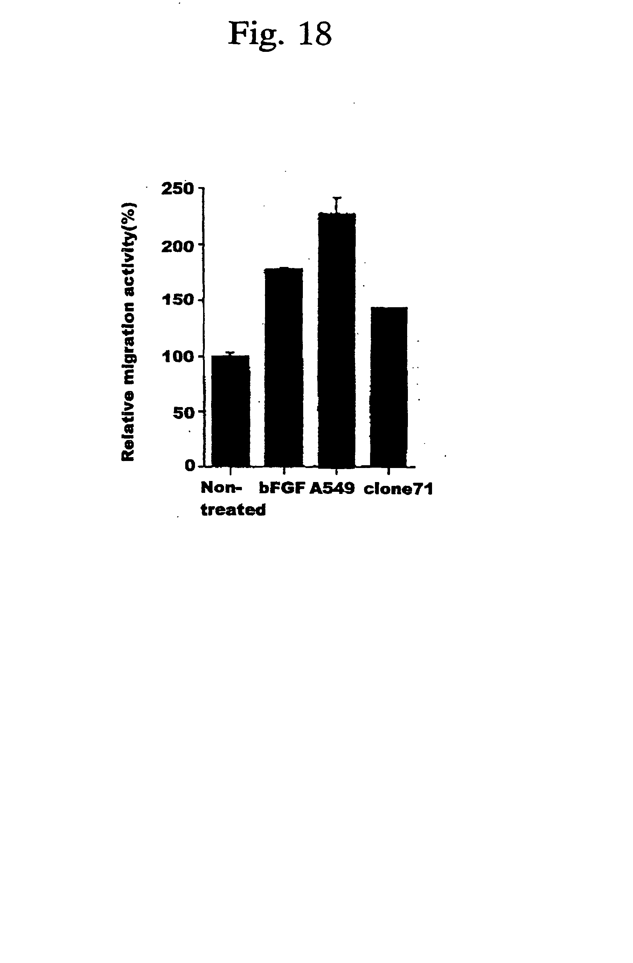

Fig. 18 is a graphical representation showing the results of the experiment shown in Fig. 16. In Fig. 18, the ordinate represents a (%) migration activity relative to a non-treated case, and the abscissa represents a non-treated case, a case of bFGF, a case of A549 cell and a case of MEF gene overexpressing cell (clone 71) from the left respectively.

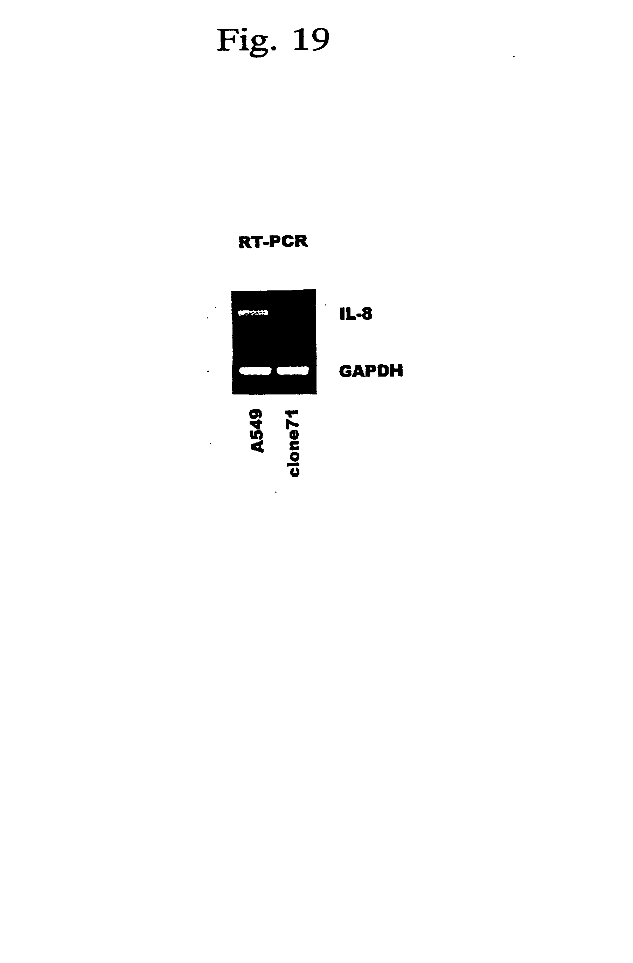

Fig. 19 is a photograph in place of a drawing, showing results of expression of IL-8 mRNA in tumors of nude mice as measured by RT-PCR. In Fig. 19, GAPDH is a control for identification.

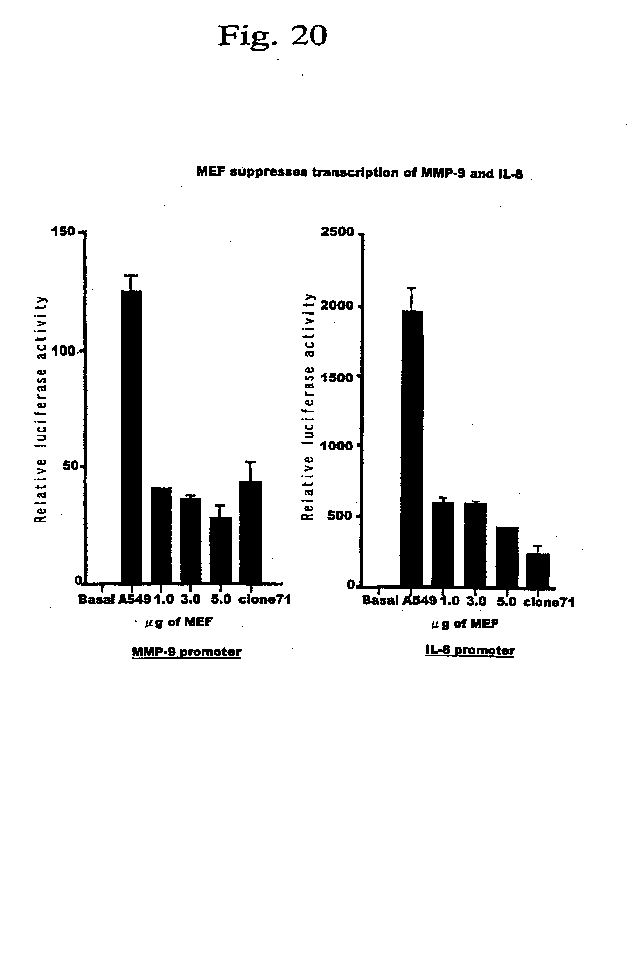

Fig. 20 shows results of performing luciferase assay using MMP-9 and IL-8 promoters. In Fig. 20, the left graph shows a case of using MMP-9 promoter, and the right graph shows a case of using IL-8 promoter. In each graph, the ordinate represents a relative luciferase activity, and the abscissa represents Basal, A549 cell, a case of adding 1.0 µM MEF, a case of adding 3.0 µM MEF, a case of adding 5.0 µM MEF and a case of adding MEF gene overexpressing cell (clone 71) from the left respectively.

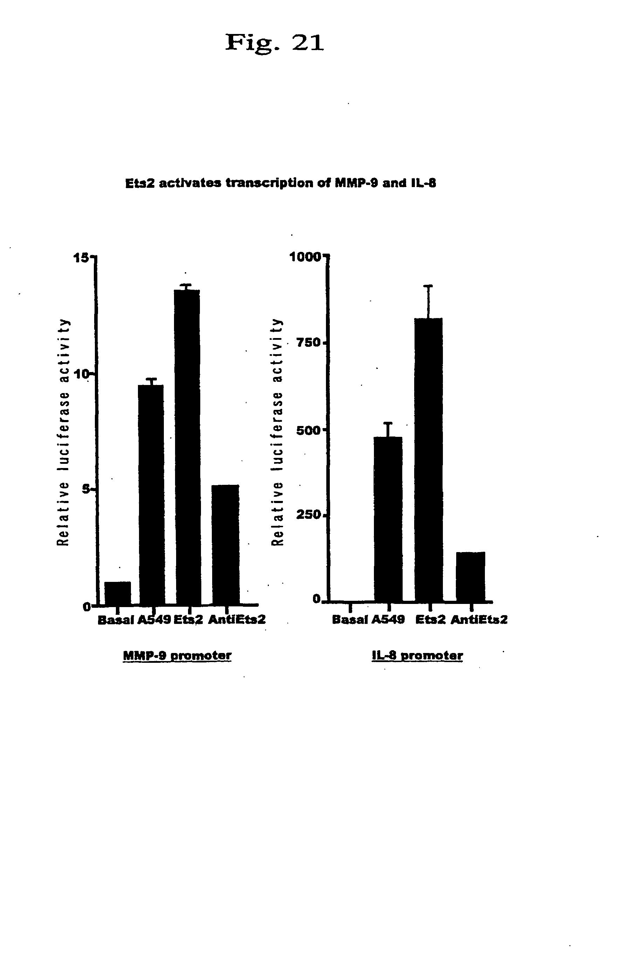

Fig. 21 is graphs showing results of performing luciferase assay using MMP-9 and IL-8 promoters. In Fig. 21, the left graph shows a case of using MMP-9 promoter, and the right graph shows a case of using IL-8 promoter. In each graph, the ordinate represents a relative luciferase activity, and the abscissa represents Basal, A549 cell, a case of adding Est-2 and a case of adding anti-Est-2 from the left respectively.

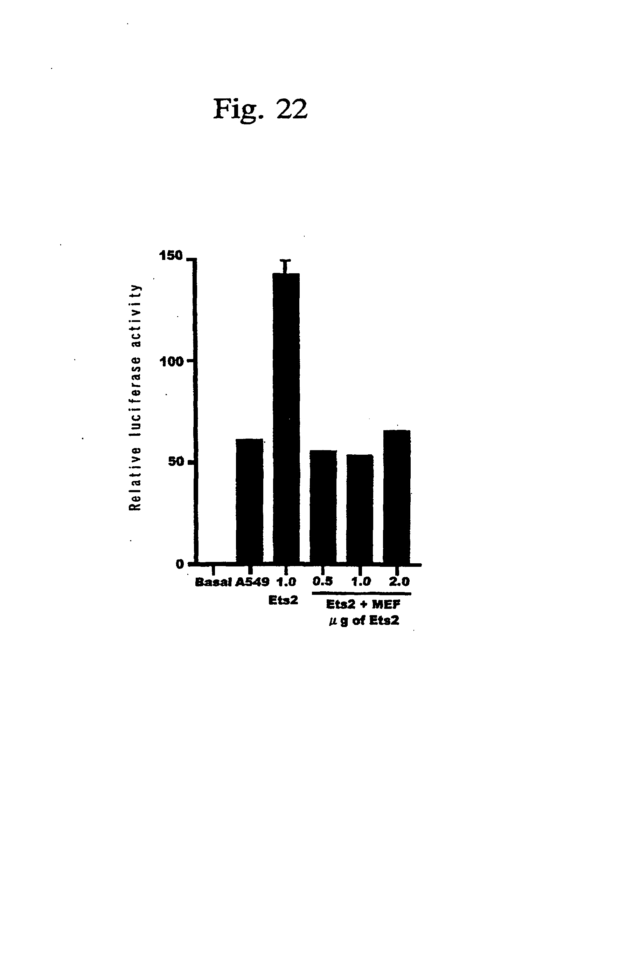

Fig. 22 shows results of performing luciferase assay in case of co-transfection of Est-2 and MEF. In Fig. 22, the ordinate represents a relative luciferase activity, and the abscissa represents Basal, A549 cell, a case of 1.0 µM Est-2, a case of adding MEF and 0.5 µM Est-2, a case of adding MEF and 1.0 µM Est-2 and a case of adding MEF and 2.0 µM Est-2 from the left respectively.

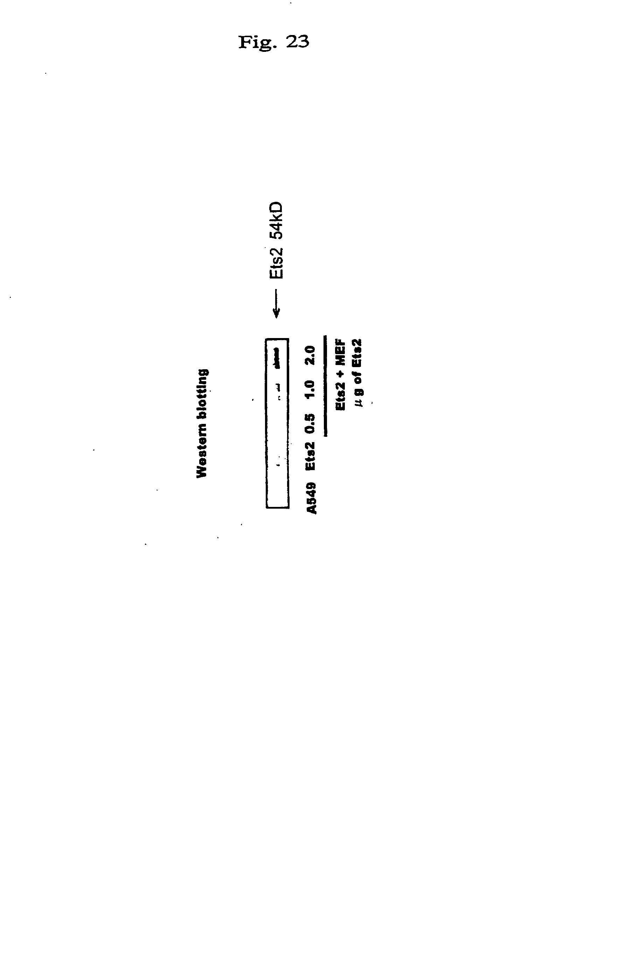

Fig. 23 is a photograph in place of a drawing, showing results of western blotting for expression of Est-2 in luciferase assay in case of co-transfection of Est-2 and MEF. Fig. 23 shows A549 cell, a case of adding 1.0 µM Est-2, a case of adding MEF and 0.5 µM Est-2, a case of adding MEF and 1.0 µM Est-2 and a case of adding MEF and 2.0 µM Est-2 from the left respectively.

### Best Mode for Carrying Out the Invention

The present inventors have assiduously conducted investigations on the ETS transcription factors, and have consequently found that cancer cells with ETS transcription factor MEF stably overexpressed showed markedly low MMP-9 activity relative to parent cells and when they are implanted in nude mice, tumor proliferation is markedly inhibited. Further, a significant function of inhibiting proliferation of synovial membrane fibroblasts has been shown.

The invention, therefore, provides the following 1 to 21.
1. A cell proliferation inhibitor comprising an ETS transcription factor or a gene encoding the same, or a substance regulating a function of the ETS transcription factor or the gene encoding the same.
2. The cell proliferation inhibitor recited in 1 above, wherein the ETS transcription factor or the gene encoding the same is an ETS transcription factor MEF protein or a gene encoding the same.
3. The expression regulator recited in 1 or 2 above, wherein the substance regulating the function of the ETS transcription factor or the gene encoding the same is a substance regulating the MEF protein or the gene encoding the same.
4. The cell proliferation inhibitor recited in 2 or 3 above, wherein the ETS transcription factor MEF protein is a protein comprising an amino acid sequence described in SEQ ID No. 1 of SEQUENCE LISTING, or the amino acid sequence in which one or more amino acids are substituted, deleted or added.
5. The cell proliferation inhibitor recited in 2 or 3 above, wherein the ETS transcription factor MEF protein or the gene encoding the same is a gene having a nucleotide sequence described in SEQ ID No. 2 of SEQUENCE LISTING, or the nucleotide sequence in which one or more nucleotides are substituted, deleted or added, or a nucleotide sequence capable of being hybridized to these nucleotide sequences under stringent conditions.
6. The cell proliferation inhibitor recited in any of 1 to 5 above, wherein the cell is an epithelial cell.
7. The cell proliferation inhibitor recited in any of 1 to 6 above, wherein the cell is a cancer cell.
8. The cell proliferation inhibitor recited in any of 1 to 6 above, wherein the cell is a synovial membrane cell.
9. The cell proliferation inhibitor recited in any of 1 to 7 above, which is an antitumor agent.
10. The cell proliferation inhibitor described in any of 1 to 6 and 8 above, which is an antirheumatic agent.
11. An MMP production inhibitor or an IL-8 production inhibitor comprising an ETS transcription factor MEF protein or a gene encoding the same, or a substance regulating a function of the MEF protein or the gene encoding the same.
12. The MMP production inhibitor recited in 11 above, wherein MMP is MMP-9.
13. A method for screening a substance capable of being a cell proliferation inhibitor, in which a cell having transfected therein a gene encoding an ETS transcription factor is used, and a test substance is added to the cell or around the cell to measure an expression amount of the gene encoding the ETS transcription factor in the cell.
14. The method recited in 13 above, wherein the gene encoding the ETS transcription factor is a gene encoding an MEF protein.
15. The method recited in 13 or 14 above, which is a method for screening a substance capable of being an active ingredient of an antitumor agent or an antirheumatic agent.
16. An antitumor agent containing a substance regulating a function of an ETS transcription factor or a gene encoding the same.
17. The antitumor agent recited in 16 above, wherein the substance regulating the function of the ETS transcription factor or the gene encoding the same is a substance regulating a function of an MEF protein or a gene encoding the same.
18. The antitumor agent recited in 16 or 17 above, wherein the substance regulating the function of the ETS transcription factor or the gene encoding the same is the substance obtained by the method recited in any of 13 to 15 above.
19. An antirheumatic agent containing a substance regulating a function of an ETS transcription factor or a gene encoding the same.
20. The antirheumatic agent recited in 19 above, wherein the substance regulating the function of the ETS transcription factor or the gene encoding the same is a substance regulating a function of an MEF protein or a gene encoding the same.
21. The antirheumatic agent recited in 19 or 20 above, wherein the substance regulating the function of the ETS transcription factor or the gene encoding the same is the substance obtained by the method recited in any of 13 to 15 above.

That is, the invention relates to a pharmaceutical composition comprising an ETS transcription factor or a gene encoding the same, or a substance regulating a function of the ETS transcription factor or the gene encoding the same, and a pharmaceutically acceptable carrier, more specifically a pharmaceutical composition useful as a cell proliferation inhibitor in an epithelial cell, a method for curing, preventing or treating diseases in which cell proliferation has to be inhibited, which comprises administering an effective amount of the pharmaceutical composition to patients suffering from diseases in which proliferation of cells of benign or malignant tumors, rheumatisms and the like has to be inhibited, and the use of an ETS transcription factor or a gene encoding the same, or a substance regulating a function of the ETS transcription factor or the gene encoding the same for producing the pharmaceutical composition.

Further, the invention relates to an antitumor agent or an antirheumatic agent, more specifically an epithelial cell antitumor agent or an antirheumatic agent, containing an ETS transcription factor or a gene encoding the same, or a substance regulating a function of the ETS transcription factor or the gene encoding the same.

Still further, the invention relates to an MMP production inhibitor, more specifically an MMP-9 production inhibitor, and an IL-8 production inhibitor, comprising an ETS transcription factor or a gene encoding the same, or a substance regulating a function of the ETS transcription factor or the gene encoding the same.

Furthermore, the invention relates to a method for screening a substance capable of being a cell proliferation inhibitor, in which a cell having transfected therein a gene encoding an ETS transcription factor is used, and a test substance is added to the cell or around the cell to measure an expression amount of the gene encoding the ETS transcription factor in the cell, and a substance capable of inhibiting cell proliferation, preferably epithelial cell proliferation which substance has been screened by the method.

The ETS transcription factor or the gene encoding the same in the invention is preferably an ETS transcription factor MEF protein or a gene encoding the same.

The invention is described in detail below.

### (MEF protein and MEF gene)

MEF is a transcription factor belonging to an ETS transcription factor group isolated from mRNA of CMK being a human megakaryocyte strain by Miyazaki et al. in 1996, comprising 663 amino acids and having a molecular weight of approximately 100 kDa (Miyazaki, Y. et al., Oncogene, 13, 1721-1729, 1996). The present inventors already clarified that like a function of an ETS transcription factor PU.1 in blood cells that it performs regulation of expression of lysozyme in the blood cells, MEF performs regulation of expression of lysozyme in epithelial cells (Kai, H. et al., J. Biol. Chem., 274(29), 20098-20102, 1999). That is, the function regulated by PU.1 in the blood cells suggests a possibility of regulating the function by the ETS transcription factor MEF in epithelial cells. Accordingly, the presence or absence of apoptosis, an MMP activity, angiogenesis and tumor proliferation were examined in a cell strain obtained by stably overexpressing MEF in epithelial cell-type cancer cells and implanting the resulting cells in nude mice.

First, a cell proliferation inhibitory activity was examined.

Results of testing a cell proliferation activity under a serum condition or a serum-free condition on pulmonary epithelial cancer-derived cell strain A549 and MEF overexpressing cell strain are shown in Fig. 1. The left graph in Fig. 1 shows the results under a 10%-serum (calf serum) condition, and the right graph in Fig. 1 shows the results under a serum-free condition. In Fig. 1, a white square mark (□) indicates a case of A549 strain as a parent cell, and a white diamond mark (◇) indicates MEF overexpressing cell strain. In the graph, the ordinate represents ABS, and the abscissa represents a culture day.

Consequently, a significant difference in proliferation activity was not observed under a serum condition, whereas significant inhibition of cell proliferation was observed in MEF overexpressing cell strain under a serum-free condition.

Moreover, regulation of MEF expression in normal cells and cancer cells was examined. Expression of MEF in human cancer cells and normal cells was analyzed by the RT-PCR method. The results are shown in Fig. 2 by a photograph in place of a drawing. GAPDH in Fig. 2 is shown as a control. In Fig. 2, HEK293 cell obtained from a human normal renal cell, A549 cell, Caco-2 cell, NCIH292 cell and Hela cell are shown from the left side.

As a result, MEF was expressed quite highly in HEK293 cell obtained from the human normal renal cell. However, in the other cancer cells, the expression amount of MEF was quite small in comparison to HEK293 cell as the normal cell, or it was an amount which could hardly be detected, based on the data standardized by the expression amount of GAPDH mRNA.

A CpG-rich region was found around exon 1 and a promoter region. Accordingly, the present inventors studied whether or not the low expression amount in cancer cells is attributed to methylation of MEF gene. For examining methylated CpG in this region, genomic DNAs from A549, Caco-2, NCIH292 and Hela cell were digested with Hpa II and Msp I sensitive to methylcytosine and resistant to isoschizomer (isoenzyme). Amplification was performed by PCR using primers capable of amplifying exon 1 containing CpG region (island). It was only A549 cell with a small expression amount of MEF, not other cells, that was methylated in a region from +500 to +1,500. It has been already reported that DNA methyl transferase (DNMT-1) is highly expressed in A549 cell and an antisense of DNMT-1 mRNA inhibits a p21 transcription activity and cell growth in A549 cell [Knox et al., 2000; Milutinovic et al., 2000].

Next, since methylcytosine is recognized with methylated DNA bound to protein MeCP2 interacting with HDAC [Ng et al, 1999], the present inventors examined the effect given by the combined use of 5-aza-2'-deoxycytidine (5-azacytidine) as a demethylation agent and tricostatin A as a histone deacylase inhibitor in MEF expression of A549 cell. The results are shown in photographs in place of drawings in Fig. 3. Fig. 3 shows the results of measuring the expression amount of MEF under various conditions by the RT-PCR method as in Fig. 2. GAPDH is shown as a control. In Fig. 3, HEK293 cell, A549 cell and Caco-2 cell are shown from the left side in an upper panel, and A549 cell and Caco-2 cell from the left side in a lower panel. In Fig. 3, DMSO indicates dimethyl sulfoxide, 5-AC indicates 5-azacytidine and TSA indicates tricostatin A respectively. As shown in Fig. 3, the combined use of 5-azacytidine (1 µM) and tricostatin A (300 nM) (right end of each cell in the lower panel in Fig. 3) clearly increased the MEF expression in A549 cell. However, the single use of these reagents increased the MEF expression only slightly. These results are also backed up by the recent finding that the methylation pattern is maintained by a dynamic balance of methylation and demethylation and a localized state of histone acetylation [Cervoni et al., 2001]. The same effect was observed in Caco-2 cell, but not in HEK293 and Hela cells. The low MEF expression in Hela cell is considered to be attributed to any other unknown mechanism. This is also backed up by the report that Hela cell lacking in known methylation-dependent inhibitor MeCP2 becomes a silence methylation gene using another route including MBD2 [Ng et al., 1999]. Accordingly, these findings show that the methylation of MEF gene is related with the low MEF expression in A549 cell and Caco-2 cell.

When MEF is an antioncogene, MEF gene overexpression can suppress tumor formation of A549 cell in which MEF is expressed only slightly. Thus, for examining in-vitro characteristics of A549 cell and MEF gene overexpressing cell, A549 cell stable transfectant (this is called clone 71) was prepared. This A549 cell stable transfectant expresses MEF.

Fig. 4 shows in-vitro characteristics of MEF gene overexpressing cell line. An upper panel in Fig. 4 shows a morphological condition by a photograph in place of a drawing. A lower panel in Fig. 4 shows F-actin staining. In Fig. 4, A549 cell, MEF gene overexpressing A594 cell and A549 cell treated with BB94 (10 µM) are shown respectively from the left side.

As a result, when the appearance of the cell was observed, the MEF gene overexpressing cell lost strong cell-cell contact to induce a morphological change. Further, as shown in Fig. 4, it was observed that F-actin was localized in an interface between MEF gene overexpressing cells, which indicates that the MEF gene overexpression expedited formation of a functional contact point to suppress mobility of cells. Further, when A549 cell was incubated in a medium containing 5-azacytidine (10 µm), the same morphological change as in MEF gene overexpressing cell was induced. Moreover, it has been reported that metastasis from epithelium to mesenchyme is induced by lack of E-cadherin or activation of MMPs [Llorens et al., 1998], expression of MMPs and E-cadherin was next examined. The right side in Fig. 5 is a photograph in place of a drawing, showing results of analyzing an MMP-9 protein content of A549 cell and MEF gene overexpressing cell in a serum-free culture supernatant (conditioned media) by western blotting using anti-MMP-9 antibody, and the left side in Fig. 5 is a photograph in place of a drawing, showing results of gelatin-zymography of MMP-9. As shown in the western blotting analysis and the gelatin zymography of Fig. 5, the MMP-9 expression was decreased in MEF gene overexpressing gene, but the E-cadherin mRNA level was unchanged. In order to examine the decrease in MMP-9 activity in MEF gene overexpressing cell, A549 cell was treated with BB94 as an MMP inhibitor. As shown in Fig. 4, BB94 (10 µM) induced typical morphological change to cause localization of F-actin in the interface between cells such as MEF gene overexpressing cells. Generally, such a morphological change thereof indicates reduction of an invasion activity. Accordingly, for examining an invasive function of A549 cell and MEF gene overexpressing cell, matrigel invasion assay was performed. The outline of the experiment is shown in Fig. 6. In an upper chamber of Fig. 6, 2.5 x 10⁵ A549 cells (left side), MEF gene overexpressing cells (center) and A549 cells (right side) treated with BB94 were inoculated respectively, and the numbers of cells on the lower side which were passed through a 8-µm filter were counted. The numbers of cells were shown in Fig. 7 in terms of a percentage (%) relative to a control (A549 cells). Further, the conditions of the respective cells are shown in Fig. 8 by photographs in place of drawings. In Fig. 8, A549 cell (left side), MEF gene overexpressing cell (center) and A549 cell treated with BB94 (right side) are shown from the left side respectively. As shown in Figs. 7 and 8, A549 cell showed a high invasion activity, while MEF gene overexpressing cell showed the invasion activity as low as the inhibitory effect of BB94.

Moreover, it has been reported that BB94 suppresses cell growth of cancer cells by inhibition and/or suppression of processing of various growth factors induced by MMPs [Bergers et al., 2000; Mira et al., 1999]. Accordingly, the influence of BB94 (10 µm) on in-vitro cell growth of A549 cells was examined. The cell growth rate of MEF gene overexpressing cells under a serum-free condition was lower than that of A549 cells. This is presumably influenced by MMP inhibition.

In view of the foregoing facts, it was shown that MEF influences the morphological change, the invasion activity and the cell growth by suppressing the expression of MMP-9 or other MMPs.

Next, it was examined whether MEF suppresses activities of A549 cell transformation and tumor formation in vivo or in vitro. Evaluation of colony formation in an agar medium revealed that MEF gene overexpressing cells did not form colonies but formed A549 cells. This indicates that MEF suppresses anchorage-dependent proliferation known as a marker of a malignant tumor.

Next, the in-vivo influence on tumor formation was examined. The foregoing A549 strain and MEF gene overexpressing cell strain were inoculated subcutaneously in the backs of nude mice, and the mice were bred in a sterile atmosphere. Two months later, sizes of tumors were compared. The results are shown in Fig. 9 by a photograph in place of a drawing. The mouse on the left side of Fig. 9 was inoculated with A549 strain, and the mouse on the right side was inoculated with MEF gene stable overexpressing cell strain. An arrow portion in Fig. 9 indicates a tumor. After 2 months from the administration, the tumor of A549 cell was grown to a diameter of about 2 cm, whereas the tumor of MEF gene overexpressing cell was grown to a diameter of less than 0.4 cm. The sizes of the respective tumors are graphically represented in Fig. 10. In Fig. 10, the ordinate represents a volume (mm²) of the tumor. The left side shows A549 cell, and the right side shows MEF gene overexpressing cell. Consequently, it was found that the tumor formation of the nude mouse with MEF gene stable overexpressing cell strain inoculated was dramatically suppressed in comparison to the nude mouse with A549 inoculated.

Further, with respect to these strains, the influences on MMP production and activity were examined. A549 strain and MEF gene stable overexpressing cell strain were inoculated in nude mice. After 2 months, tumor nodes formed subcutaneously were recovered from the nude mice. The MMP production in these tissues was examined by gelatin zymography and western blotting. The results are shown in Fig. 11 by photographs in place of drawings. In Fig. 11, an upper panel shows the results of gelatin zymography, and a lower panel shows the results of western blotting using anti-MMP-9 antibody in a usual manner. In Fig. 11, the left side shows a case of A549 strain, and the right side shows a case of MEF gene stable overexpressing cell strain. Each case shows three examples.

Consequently, it was found that the MMP-9 activity was markedly suppressed in the tumor of the nude mouse with MEF gene stable overexpressing cell strain inoculated. It was further found that the MMP-9 expression was markedly suppressed in the tumor of the nude mouse with MEF gene stable overexpressing cell strain inoculated.

In addition, these cells were subjected to hematoxylin-eosin (HE) staining. The results are shown in Fig. 12 by photographs in place of drawings. In Fig. 12, an upper panel shows the results of HE staining, and a lower panel shows the results of lysozyme. As a result, the tumor of A549 cell was incompletely differentiated, whereas the tumor of MEF gene overexpressing cell was fully differentiated into a gland with many central cavities (Fig. 12). The present inventors indicated previously that MEF was up-regulated by expression of lysozyme of A549 cell as a marker of gland serous cell differentiation [Kai, 1999]. According to this, the overexpression of lysozyme was found only in the central cavity of the MEF gene overexpressing tumor (lower panel in Fig. 12). Thus, induction of differentiation of an epithelial cell by MEF is related with suppression of in-vivo tumor formation by A549 cell.

Next, an influence on angiogenesis in tumor tissues with A549 strain and MEF gene stable overexpressing cell strain implanted was examined.

That is, A549 strain and MEF gene stable overexpressing cell strain were inoculated in nude mice. After 2 months, tumor nodes formed subcutaneously were recovered from the nude mice, and freeze-stored. The stored tissues were cut into sections having a thickness of 10 µm to produce tissue specimens. These were stained by the HE staining method. The results are shown in Fig. 13 by photographs in place of drawings. In Fig. 13, the left photograph is a case of A549 strain, and the right photograph is a case of MEF gene stable overexpressing strain (No. 71).

Consequently, it was found that angiogenesis was markedly suppressed in the nude mouse with MEF gene stable overexpressing cell strain (No. 71) inoculated.

Next, induction of apoptosis was examined.

Apoptosis in the foregoing tissue specimens was detected using an apoptosis detection kit. The results are shown in Fig. 14 by photographs in place of drawings. In Fig. 14, the left photograph is a case of A549 stain, and the right photograph is a case of MEF gene stable overexpressing cell strain (No. 71).

Consequently, it was found that induction of apoptosis was significantly observed in the tumor of the nude mouse with MEF gene stable overexpressing cell strain.

Moreover, an influence on a function of proliferating synovial membrane fibroblasts was examined using a rabbit.

A function of inhibiting synovial membrane cell proliferation was examined by the MTT method using synovial membrane fibroblasts prepared by outgrowth method according to the Werb and Burleigh (1974) method from a knee joint synovial membrane tissue extracted from a Japanese white rabbit (body weight approximately 3 kg). The results are shown in Table 1 below.

**Table 1**

| Cell proliferation rate of MEF-transfected cell and MEF-untransfected cell | | |
|---|---|---|
| | Cell proliferation rate | |
| | After 48 hours from transfection | After 72 hours from transfection |
| MEF-untransfected synovial membrane fibroblast | +++ | +++ |
| MEF-transfected synovial membrane fibroblast | ± | ± |

As a result, it was found that cells with MEF transfected had the marked function of inhibiting synovial membrane cell proliferation.

These results revealed that the induction of apoptosis was observed in the cell strain with MEF transfected and the marked decrease in MMP production and activity, the inhibition of angiogenesis and the inhibition of tumor proliferation were observed in comparison to the parent cell strain. Accordingly, it was found that the excellent effect of inhibiting cell proliferation was exhibited by transfecting and expressing the ETS transcription factor MEF in cells derived from diseases.

Moreover, for examining whether MEF influences angiogenesis of tumors, angiogenesis of nude mice was analyzed by an immunological staining method with CD31 as a marker of vessel epithelial cells. The numbers of medium and large vessels in the MEF gene overexpressing tumor were clearly decreased by 75% and 31% respectively relative to the control. The results are shown in Fig. 15. In Fig. 15, the ordinate represents a value (%) relative to the number of vessels of A549 cell, and the abscissa represents the vessel size of 50 mm or more and the vessel size of 50 mm or less. In each size, the left side shows a case of A549 cell, and the right side shows a case of MEF gene overexpressing cell (clone 71).

Successively, it was further examined whether MEF gene overexpression of A549 cells influences metastasis of vessel endothelial cells. An outline of an experiment is shown in Fig. 16. In Fig. 16, a small black circle shows an angiogenetic factor, and an elliptic black circle shows a human umbilical vein endothelial cell (HUVEC). Human umbilical vein endothelial cells (HUVECs) were inoculated in an upper chamber, and cells migrating in a medium of a lower chamber were observed through a 8-µm filter.

The results are shown in Fig. 17 by photographs in place of drawings. The left upper photograph shows a non-treated case, the right upper photograph shows a case of adding bFGF, the left lower photograph shows a case of a medium of A549 cell, and the right lower photograph shows MEF gene overexpressing cell (clone 71). A graphical representation of the results is shown in Fig. 18. In Fig. 18, the ordinate represents a (%) migration activity relative to a non-treated case, and the abscissa represents a non-treated case, a case of bFGF, a case of A549 cell and a case of MEF gene overexpressing cell (clone 71) from the left side respectively. In a serum-free culture supernatant (conditioned media) of A549 cell, migration of human umbilical vein endothelial cells (HUVECs) was accelerated, whereas the same effect was not found in the serum-free culture supernatant of MEF gene overexpressing cell. In a positive control, basic FGF (bFGF) increased migration of HUVECs (Figs. 17 and 18).

It has been reported that gland proliferation in A549 tumor is induced by blocking an IGF-1 signal system [Jiang, 1999]. Various growth factors are found in a substrate matrix, and bound to various binding proteins and extracellular matrixes [Bergers, 2000]. It has been reported that MMPs participate in not only invasion of tumor but also in treatment and release of various growth factors such as VEGF and IGF-1 [Bergers, 2000; Mira, 1999]. Accordingly, MMPs are required to unit these growth factors, and indirectly related with tumor growth and angiogenesis. For identifying that these factors are related with an MEF function of tumor inhibition, the present inventors have first focussed on expression of MMPs. The reason is that especially expression of VEGF and IGF-1 in A549 cell is not performed by MEF gene overexpression. According to gelatin zymography, expression of MMP-9 and MMP-2 was considerably reduced in MEF gene overexpressing tumors. This result of MMP-9 was also identified by western blotting analysis and immunohistochemistry. MEF inhibited expression of MMP-2. Although an MMP-2 promoter presumably free from an ets consensus motif is not directly controlled by an ETS transcription factor, inhibition of MMP-2 greatly contributes toward suppression of malignant tumors. One reason is based on the finding that the suppression of MMP-2 alone inhibits metastasis from a prevascular state to a vessel in tumorigenesis and then inhibits tumor growth.

The angiogenesis is induced by proliferation and migration of blood endothelial cells, and it is accelerated by an angiogenetic factor in case of A549 cells. IL-8 is a strong angiogenetic factor [Arenberg, 1996]. Recently, it has been reported that PEA3 is related with angiogenesis of tumors by induction of IL-8 [Arenberg, 1996]. It has been considered that MEF might suppress angiogenesis of tumors by inhibiting expression of IL-8. Immunohistochemical analysis in tumors of nude mice has indicated that expression of IL-8 is decreased in MEF gene overexpressing tumors (Fig. 19). In the in-vitro incubation, the expression of IL-8 mRNA was considerably reduced in MEF gene overexpressing cells in comparison to A549 cells (Fig. 19). Fig. 19 is a photograph in place of a drawing, showing results of expression of IL-8 mRNA as measured by RT-PCR. In Fig. 19, GAPDH is a control for identification.

These results suggested that MEF suppresses transcription of IL-8 whereby the angiogenesis of tumors is inhibited at least partially.

Accordingly, the present inventors further examined the function of MEF on transcription of MMP-9 and IL-8. For examining whether MEF directly suppresses transcription of MMP-9 and IL-8, the present inventors performed luciferase assay using MMP-9 and IL-8 promoters. The results are shown in Fig. 20. In Fig. 20, the left graph shows a case of using MMP-9 promoter, and the right graph shows a case of using IL-8 promoter. In each case, the ordinate represents a relative luciferase activity, and the abscissa represents Basal, A549 cell, a case of adding 1.0 µM MEF, a case of adding 3.0 µM MEF, a case of adding 5.0 µM MEF and a case of adding MEF gene overexpressing cell (clone 71) from the left respectively. In A549 cell, a high luciferase activity of MMP-9 and IL-8 promoters was observed (Fig. 20). MEF reduced the luciferase activity of these promoters dependently on the concentration. Meanwhile, among ETS transcription factors (ELF-1, ETS-1, PEA3 and ESE-1), only ETS-2 increased the luciferase activity of MMP-9 and IL-8 promoters, and the antisense of ETS-2 mRNA decreased the luciferase activity of these promoters (Fig. 21). Fig. 21 shows results of performing luciferase assay using MMP-9 and IL-8 promoters. In Fig. 21, the left graph shows a case of using MMP-9 promoter, and the right graph shows a case of using IL-8 promoter. In each graph, the ordinate represents a relative luciferase activity, and the abscissa represents Basal, A549 cell, a case of adding Est-2 and a case of adding anti-Est-2 from the left respectively.

The finding of MMP-9 activation with ETS-2 is also backed up by the study on target deletion of Ets-2 using mice [Yamamoto, 1998]. Since MEF is, unlike PU-1, free from a repressor region [Kihara-Negishi, 2001] and interacts with HDAC or Sin3A having a deacetylase activity, the present inventors next assumed that MEF is a competitor with ETS-2 in ets binding sites of these promoters. Fig. 22 shows results of performing luciferase assay in case of co-transfection of Est-2 and MEF. In Fig. 22, the ordinate represents a relative luciferase activity, and the abscissa represents Basal, A549 cell, a case of adding 1.0 µM Est-2, a case of adding MEF and 0.5 µM Est-2, a case of adding MEF and 1.0 µM Est-2 and a case of adding MEF and 2.0 µM Est-2 from the left respectively. Results of western blotting for expression of Est-2 in these cases are shown in Fig. 23 by a photograph in place of a drawing.

As shown in Fig. 22, ETS2 did not activate these promoters by co-transfection with MEF. An ETS2 induction activity was inhibited even by co-transfection of an expression vector in which only an ETS domain of MEF is expressed. For further examining whether a promoter activity is inhibited by blocking ETS-2 bound to ets binding sites of the promoters, "decoy type nucleic acid" made of a double-stranded synthetic oligonucleotide comprising 3 ets binding motifs of MMP-9 and IL-2 promoters and macrophage colony stimulation factor (GM-CSF) being an important cytokine in tumorigenesis was formed. This "decoy type nucleic acid" having the ets binding motifs inhibited a promoter activity induced by ETS2 overexpression, but "decoy type nucleic acid" with variant ets binding motifs in which 5'-GGAA-3' was changed to 5'-TCAA-3' did not inhibit the same. On the basis of the foregoing finding, the effects of 5-azacytidine (1 µM) and trichosamine A (1 µM) in MMP-9 and IL-8 promoter activities were examined for identifying whether demethylation of MEF gene controls these promoter activities. The MMP-9 and IL-8 promoter activities were inhibited with 5-azacytidine and trichosamine A.

From the foregoing, it was shown that MEF is an antioncogene and this is controlled on a downstream side by methylation in cancer cells.

The foregoing fact reveals that MEF inhibits tumor formation of human non-small-cell pulmonary carcinoma A549 cell in vitro and in vivo which is attributed to inhibition of both MMPs and IL-8. The inhibition mechanism is competition with binding of ETS-2 to ets binding sites of MMPs and IL-8 promoters. Further, the MEF expression in some cancer cells is down-regulated by methylation of CpG island'around the exon 1 region of MEF gene. Accordingly, it has been considered that MEF is a novel antioncogene localized in X chromosome. This is also strongly backed up by the fact often observed in a clinical site that LOH is found in Xq 25-26.1 in ovarian cancer and breast cancer [Choi, 1997; Choi, 1998]. This is further backed up by the latest study that transcription control with MEF limits G1 phage of a cell cycle [Miyazaki, 2001]. Since MEF as an antioncogene inhibits growth of tumors, it is reasonable that MEF is inactivated during an S period of the cell cycle. Besides, expression of ESE-3 as an oncogene by grand differentiation [Tugores, 2001] considerably showed up-regulation in tumors of MEF gene overexpression. Accordingly, MEF might have a synergistic function with ESE-3 on a function of inhibiting malignant tumors. The present inventors have lately found that GM-CSF is up-regulated with ETS-2 as a target of protein kinase C. This up-regulation is inhibited also by MEF. In connection with this finding as well, that MEF acts as an antioncogene in the invention is backed up by the report that the production of GM-CSF is related with both in-vitro invasion and progression of pulmonary platycyte cancer [Tsuruta, 1998]. Further, GM-CSF can have an effect of inducing human non-small-cell pulmonary cancer. It has been moreover reported that GM-CSF activates migration of endothelial precursor cells for tumor angiogenesis [Takahashi, 1999].

Tumor progression includes steps of proliferation, angiogenesis and metastasis, and each step is controlled by a positive or negative regulation balance. For example, angiogenesis is controlled with an angiogenetic switch. Angiogenesis is switched on when a positive regulation level of VEGF, MMPs, IL-8 and bFGF (basic fibroblast growth factor) and the like is higher than a negative regulation level of thrombospondin-1 and -2, endostatin and the like [Hanahan, 1996; Bergers, 2000]. In tumor cells, it is indicated that some ETS transcription factors up-regulate transcription of the positive regulation of MMPs and IL-8 [Iguchi, 2000; Sementchenko, 2000]. Moreover, an ETS factor group activated with an oncogene factor group controls tumor progression.

Many researchers have focussed on how extracellular factors act on transformation of cells. However, it has not been clarified how a balance of intracellular factors such as transcription factors controls transformation of cells. In order to find how a balance of transcription factors in the same family controls transformation of cells, the present inventors have focussed on ETS transcription factors. The ETS family of transcription factors has an important role in growth, destruction and proliferation of cells. Many transformation-associated genes contain adjacent binding sites to both transcription factors of ETS and AP-1 families, and such an element regulates activation of transcription in a wide range of activated ontogenesis [Sharrocks, 1997]. Some ETS transcription factors are known to be a downstream target of an Ras-Raf-MEK signaling route [Wasylyk, 1998]. Acceleration of carcinogenicity of this route activates a transcription activity to change localization of ETS-1 and ETS-2 by phosphorylation of a threonine residue in a main region [Yang, 1996; Wasylyk, 1997]. ETS-2 is known to be an important mediator of cell transformation. This is because a predominant negative structure of ETS-2 can block transformation with Ras or HER2 [Foos, 1998]. Breast cancer cells are inhibited by intercourse with a heterozygote mouse that has caused target variation of ETS-2 [Neznanov, 1999]. As backed up by the invention, ETS-2 activates a malignant tumor factor group such as MMP-9 in A549 cell and IL-8. The finding of activation of MMP-9 with ETS-2 is backed up also by the study on target deletion of Ets-2 in mice [Yamamoto, 1998]. Accordingly, inhibition of ETS-2 might be a good target in molecular tumor therapy.

Recently, it has been reported that some ETS transcription factors inhibit transcription of MMPs and HER2 [Mavrothalassitis, 2000]. Accordingly, tumor. progression can be controlled by a balance of a positive or negative ETS factor group. In the invention, it has been clarified that the balance of MEF and ETS-2 in cancer cells has an important role in determining malignant tumors. It has been indicated that some ETS transcription factors are phosphorylated by Ras signal transmission, and entered into a nucleus to control transcription of oncogene. However, ESE-3 (one of ETS transcription factors) is a protein of a nucleus, and there is a recent report that it inhibits transcription of Ras signal transmission (Tugores, 2001]. Accordingly, a nuclear ETS factor group such as ESE-3 negatively regulates a cytoplasmic ETS factor group to stabilize cell homeostasis. Intracellular localization of MEF was determined under a serum or serum-free condition in the presence of predominant negative Ras and MEK using green fluorescent protein-MEF fusion protein. A549 cell has variant K-Ras [Mitchell, 1995], so that activation of a Ras-MEK-MAPK route occurs. It has been found that MEF is localized in a nucleus. ETS-2 has been localized in a cytoplasma and a nucleus in a non-stimulated state. The ETS factor group controls cell transformation. That is, MEF and ESE-3 act as a cancer suppressor substance of a carcinogenic ETS transcription factor such as ETS-2.

The present inventors reported ago that MEF activates a promoter of lysozyme in A549 cell [Kai, 1999]. In the invention, however, it has been shown that MEF suppresses MMP-9 and IL-8 promoters. That is, regulation of transcription with MEF depends on conditions of promoters and cells. Likewise, Fli-1 can function as an activator for a promoter of tenascin-C [Shirasaki, 1999]. Meanwhile, it functions as a repressor depending on a condition of a promoter of collagen [Czuwara-Ladykowska, 2001]. With respect to the up-regulation of lysozyme by MEF, the present inventors reported ago that in addition to an ets binding site (-46/-40) of a promoter in the vicinity of bovine lysozyme gene 5A, a second element (-100/-51) site exists [Kai, 1996; Kai, 1999]. This enhancer has not been identified yet, but MEF requires specific interaction with an enhancer-binding protein for activating expression of lysozyme. Anyway, interaction between ETS factors dependent on a condition of a target promoter acts as a molecular switch for inhibition and activation of genes, or vice versa.

The invention contributes toward not only cancer therapy using the "decoy type" DNA but also cancer diagnosis based on the finding of epigenetic modification of LOH, SNPs and MEF gene in cancers in clinics.

Accordingly, the invention is useful as antitumor agents of melanoma, squamous cell carcinoma, breast cancer, rectum cancer, digestive organ cancer, pulmonary cancer, large bowel cancer, uterine cancer, renal cell carcinoma, testicular carcinoma, bladder cancer, ovarian cancer, prostatic cancer, multiple myeloma, chronic myeloid leukemia, malignant lymphoma neuroblastoma, brain tumor and tumors caused by metastasis of these, as well as antirheumatic agents.

Examples of the ETS transcription factor in the invention include various transcription factors capable of recognizing an ETS binding site. Of these, MEF is preferable. MEF has an amino acid sequence described in SEQ ID No. 1. MEF available in the invention is not limited to MEF having said amino acid sequence but include any MEF that have an activity of regulating expression of at least one of GM-CSF, β-defencin-1 and β-defencin-2. Preferable is MEF having an amino acid sequence capable of recognizing an ETS binding site. Accordingly, MEF of the invention includes a polypeptide comprising an amino acid sequence in which one or more amino acids in the amino acid sequence described in SEQ ID No. 1 are substituted , or deleted, and a polypeptide comprising an amino acid sequence in which one or more amino acids are added to the amino acid sequence described in SEQ ID No. 1. These polypeptides are included in the invention so long as they are variant proteins of MEF and have the foregoing activity.

Likewise, the gene encoding the ETS transcription factor in the invention includes the foregoing gene encoding the ETS transcription factor in the invention. The gene encoding the ETS transcription factor in the invention is preferably. the foregoing gene encoding MEF of the invention. An example of the gene encoding MEF is described in SEQ ID No. 2 of SEQUENCE LISTING. However, the gene of the invention is not limited to the gene having this base sequence. For example, a polynucleotide comprising a nucleotide sequence in which in the nucleotide sequence described in SEQ ID No. 2 one or more nucleotides are substituted or deleted, or a polynucleotide comprising a nucleotide sequence in which one or more nucleotides are added to the nucleotide sequence described in SEQ ID No. 2 is also included in the gene of the invention.

Further, a gene comprising a base sequence capable of being hybridized to the gene having the foregoing base sequence under stringent conditions is also included in the gene of the invention.

A sugar chain is added to many of ordinary proteins, and such an addition can be regulated by converting one or more amino acids. A polypeptide with the sugar chain addition regulated in the amino acid sequence described in SEQ ID No. 1 is also included in the invention so long as it has the foregoing activity. Further, a polynucleotide encoding the polypeptide is likewise included in the invention.

Moreover, the invention includes disease therapeutic methods or therapeutic agents using substances such as ETS transcription factors in epithelial cells, preferably MEF proteins or proteins, peptides, organic compounds and steroids enhancing expression of MEF gene.

### (Gene therapy)

The invention can be used in gene therapy against the various diseases listed above by incorporating MEF into a therapeutic vector.

In the invention, the vector used in gene therapy includes but not limited to, vectors derived from recombinant vaccine virus, poliovirus, influenza virus, adenovirus, adeno-associated virus, herpesvirus, HIV virus, Sendai virus and the like. Further, sequences of appropriate promoters, replication origins, selected markers, RNA splicing sites, polyadenylation signals and the like related with gene expression are introduced in the vectors.

The invention is used as gene therapeutic agents in a usual manner by incorporation into the vectors. That is, in case of performing gene therapy, it is advisable that the recombinant virus vector is contacted with target cells in therapy or inserted into an expression vector such as a plasmid vector to transfect the same into target cells. The transfection can then be performed by a known method such as a calcium phosphate method, a liposome method, an electroporation method or a DEAE-dextran method.

The term "oligonucleotide" used in the present specification means an oligonucleotide formed from a naturally occurring base and a sugar moiety bound by an inherent phosphodiester bond, and its analogues. Accordingly, the first group encompassed within the term is includes naturally occurring species or synthetic species generated from naturally occurring subunits or homologues thereof. It refers to a base-sugar combination bound to subunits through a phosphodiester bond or other bond. A second group of the oligonucleotide is analogues thereof which function similarly to the oligonucleotide but have residues with a moiety never occurring naturally. These include oligonucleotides with phosphate groups, sugar moieties and 3'- and 5'-terminals chemically modified for enhancing the stability. Examples thereof include oligophosphorothioate where one of oxygen atoms in a phosphodiester group between nucleotides is substituted with sulfur and oligomethyl phosphonate where it is substituted with -CH₃. The phosphodiester bond may be replaced with other nonionic and achiral structure. As for oligonucleotide analogues, species including modified bases, namely, purines and pyrimidines other than those usually found in nature, may be used. Such oligonucleotides are also included in the invention as the DNA derivatives so long as they exhibit the same function as the antisense DNA of the invention.

In the invention, the target portion of mRNA to which the oligonucleotide is hybridized is preferably a transcription initiation site, a translation initiation site, an intron-exon binding site or a 5'-cap site. In consideration of a secondary structure of mRNA, a site free from steric hindrance has to be selected.

### (Production and use of MEF)

MEF of the invention can be produced by transforming host cells such as procaryotic cells or eucaryotic cells with an expression vector having transfected therein DNA described in SEQ ID No. 2 and sequences of promoters, a replication origin, a selected marker, an RNA splicing site and a polyadenylation signal appropriate for the vector and related with gene expression and expressing MEF gene in the host cells. Further, the MEF of the invention can be produced by ligating a gene encoding a different protein to the DNA relating to the invention to allow the expression of a fusion protein to expedite purification of MEF, increase the amount expression, or to carry out an appropriate treatment at the purification step to excise the generated MEF.

Further, mutant MEF can also be produced by mutation of one or more nucleotides of DNA described in SEQ ID No. 2, adding another nucleotide thereto, cleaving a part of 3'-side or 5'-side or removing one or more midway nucleotides.

For this purpose, procaryotic host cells among hosts used in the expression system include Escherichia coli and Bacillus subtilis. Host cells of eucaryotic microorganisms among eucaryotes include yeast and myxomycetes. Instead, insect cells such as Sf9 may also be used as host cells. Further, the host cells derived from animal cells include COS cell and CHO cell.

In the invention, MEF produced by the above method can be used after separation from the inside or outside of the host cells and purification. For separation and purification of MEF, the common methods for separating and purifying the proteins can be used. The methods such as various kinds of chromatographies, ultrafiltration, salting, dialysis can be selected and used in combination upon requirement.

In the invention, MEF can be administered by intravenous administration, local administration to the affected part, oral administration or the like. In the administration, MEF is formulated into preparations appropriate for the administration by adding thereto pharmaceutically acceptable additives such as carriers, excipients, stabilizers and solubilizers.

In accordance with the invention, furthermore, an antibody recognizing an oligopeptide having at least five sequential amino acids in the amino acid sequence (SEQ ID No:1) of MEF can be prepared. Specifically, the antibody can be obtained by immunizing an animal with an oligopeptide as an antigen, collecting the antibody generated in vivo and then purifying the antibody. The antibody includes polyclonal antibody and monoclonal antibody, and methods for purifying these antibodies are known to those skilled in the art. Any anti-MEF antibodies obtained in such a manner can be used for detection and quantitative determination of MEF in the various immunological assays such as enzyme immunoassay e.g. ELISA, radio-immunoassay, and fluorescence immunoassay, or for MEF purification on columns.

As the active ingredient of the invention, not only the ETS transcription factor or the gene encoding the same but also the substance regulating the function of the ETS transcription factor or the gene encoding the same can give the same results in vivo or in vitro. Thus, the substance regulating the function of the ETS transcription factor or the gene encoding the same is also available.

As the substance regulating the function of the ETS transcription factor or the gene encoding the same, a substance capable of regulating the expression of the ETS transcription factor or the gene encoding the same in vivo or in vitro can be used. For example, substances capable of regulating the expression of the same, such as proteins, peptides, organic compounds and steroids, are available. Further, "regulating the function" in the method of the invention means inhibiting the function or enhancing the function or both inhibiting and enhancing the function in some conditions. Preferably, the substance is a substance having an action of inhibiting or enhancing the function.

The invention provides a method for screening these substances. That is, the invention provides a method for screening a substance capable of being a cell proliferation inhibitor, in which a cell having transfected therein a gene encoding an ETS transcription factor is used, and a test substance is added to the cell or around the cell to measure an expression amount of the gene encoding the ETS transcription factor in the cell, and a substance capable of controlling cell proliferation which substance is screened by the method.

As the cell having transfected therein the gene encoding the ETS transcription factor in the invention, a cell having a gene encoding the naturally occuring ETS transcription factor may be used as it is. Preferably, a transformant obtained by transfecting a gene encoding an ETS transcription factor into an appropriate expression vector and subsequently introducing the resulting vector into an epithelial cell or various microbial cells can be used. The microbial cells used which can be used in this method are, procaryotic host cells or host cells of eucaryotic microbes.

According to the method of the invention, it is possible to identify the substance whether it has the same objet or not, by adding a test substance of various concentrations to or around a cell transfected therein a gene encoding the ETS transcription factor, and measuring the amount of the ETS transcription factor, or GM-CSF and/or the defensin protein expressed therein.

The substance obtained by this method of the invention is a substance which can inhibit cell proliferation in cells, preferably epithelial cells. Thus, it can inhibit cell proliferation. More specifically, the substance is a substance useful as a therapeutic agent, a preventing agent or a treating agent of the foregoing diseases such as benign or malignant tumors and rheumatisms. Accordingly, the invention includes the substance capable of inhibiting cell proliferation which substance is obtained by this method.

Further, the invention includes a pharmaceutical composition of a therapeutic agent, a preventing agent or a treating agent, containing the substance screened by this method as an active ingredient, and a therapeutic method of diseases requiring inhibition of cell proliferation, such as benign or malignant tumors and rheumatisms, and the use for production of the pharmaceutical composition.

Still further, the invention provides an MMP production inhibitor or an IL-8 production inhibitor comprising an ETS transcription factor MEF protein or a gene encoding the same, or a substance regulating a function of the MEF protein or the gene encoding the same. As MMP, MMP-9 is preferable. IL-8 has been known to be a potent angiogenetic factor [Arenberg, 1996], and angiogenesis is inhibited by inhibiting production of IL-8. It is considered that not only the angiogenetic function of IL-8 but also various functions caused by IL-8 can also be inhibited by the function of inhibiting IL-8 production in the invention.

### Examples

The invention is illustrated more specifically below by referring to Examples. However, the invention is not limited by these Examples. Origins of reagents are described for convenience sake, and do not limit the invention.

Human non-small-cell pulmonary carcinoma-derived A549, human fetal liver-derived cell HEK293 and human large bowel cancer-derived Caco-2 were procured from ATCC (American Type Culture Collection). Cells were suspended in 10% fetal bovine serum-containing DMEM (containing 100 U/ml·penicillin and 0.1 g/l·streptomycin), inoculated in a culture flask and then subjected to subculture in 5% CO₂ at 37°C.

### Example 1

### (Production of MEF gene stable overexpressing cell strain)

### 1. Incubation of human pulmonary epithelium-derived A549 cell strain

Human pulmonary epithelial cell strain A549 was statically incubated in a cell proliferation culture solution obtained by adding 10% FBS (fetal bovine serum)(Hyclone, Lot No. AGB 6235) and antibiotics (penicillin G (100 units/ml) and streptomycin (100 µg/ml) to a basic culture solution (Dulbecco's Modified Eagle Medium, pH=7.4) in 5% CO₂ at 37°C.

### 2. Preparation of a gene

100 µg of an expression vector (pCB6) having MEF cDNA incorporated therein was digested with restriction enzyme ApaL I to prepare linear DNA.

### 3. Transfection of MEF gene into cells

Cells in a subconfluent state (from 50 to 60%) were recovered by trypsin digestion, and suspended in a site mix solution (120 mM KCl, 0.15 mM CaCl₂, 10 mM K₂HPO₄/KH₂PO₄, pH 7.6, 25 mM Hepes, pH 7.6, 2 mM EGTA, pH 7.6, 5 mM MgCl₂, 2 mM ATP, pH 7.6; 5 mM glutathione; pH adjusted with KOH) containing the foregoing DNA (100 µg). The suspension was then poured into cuvettes (ELECTROPORATION CUVETTES PLUSTM, 2 mm gap, BTX), and allowed to stand on ice for 10 minutes. Subsequently, electric shock (500 V, 1,350 mF) was applied with a pulser (ELECTRO CELL MANIPURATION ECM 600, BTX), and the resulting suspension was again allowed to stand on ice for 10 minutes, and incubated in 5% CO₂ for 37°C. Incidentally, the amount of DNA used was measured from an absorbance of UV (260 nm), and DNA having a purity of 85% or more was used.

### 4. Determination of MEF gene stable overexpressing strain

When the cells became nearly 50%-confluent, G418 (Nacalai Tesque) was added to a final concentration of 1.0 mg/ml, and the mixture was further incubated for one week. Incidentally, G418 was used at a concentration at which untransfected cells were destroyed in one week. One week later, colonies of survival cells were recovered, and incubated in separate 60-mm culture dishes, and the selection with G418 was conducted again. Finally, the expression of MEF gene in each cell was identified by northern blotting analysis and RT-PCR to obtain MEF gene stable overexpressing cell strain (No. 71 strain).

### Example 2 (Cell proliferation inhibitory activity)

Pulmonary epithelial cancer-derived cell strain A549 and MEF overexpressing cell strain produced in Example 1 were inoculated in a medium (Dulbecco's Modified Eagle Medium) containing or not containing 10% serum (fetal bovine serum) at a concentration of 1 x 10⁴ cells/ml, and incubated in 5% CO₂ at 37°C for 24 hours. The number of cells with the lapse of time after the inoculation was measured by the MMT method.

The MMT method was conducted as follows. First, to the cells 4 hours before the measurement were added a phosphate buffered saline solution (PBS (-) solution) of MMT (3-(4,5-dimethylthiazol-2-yl)-2,5-diphenyltetrazolium bromine) and, per well of a 96-well microplate, 100 ml of hydrochloric acid (0.04 N) isopropanol and 20 ml of a 3% SDS aqueous solution to dissolve formazan formed by action of mitochondria of viable cells. Then, an absorbance at 595 nm (control 655 nm) of the solution was measured. The results are shown in Fig. 1.

Consequently, no marked difference in proliferation activity was found under the serum condition, whereas the marked inhibition of cell proliferation was observed in MEF overexpressing cell strain under the serum-free condition.

### Example 3 (Influence on tumor formation)

A549 and MEF gene stable overexpressing cell strain were suspended in PBS (-) solutions at concentrations of 1 x 10⁶ cells/100 µl, and subcutaneously inoculated in the backs of nude mice, and the nude mice were bred in a sterile atmosphere. Two months later, the sizes of tumors were compared. Photographs indicating the results are shown in Fig. 2.

Consequently, the tumor formation in the nude mouse inoculated with MEF gene stable overexpressing cell strain was remarkably suppressed in comparison to the nude mouse inoculated with *A549.*

### Example 4 (Influence on MMP production and activity)

Two months after the inoculation of A549 and MEF gene stable overexpressing cell strain, tumor nodes formed subcutaneously in the nude mice were recovered. Each thereof was dipped in an equal amount of a lysis buffer (20 mM Tris-HCl, 0.1 M NaCl, 0.5% Triton-X, pH 7.4), and allowed to stand overnight at 4°C. A supernatant was then recovered. The resulting supernatant was suspended in an SDS sample buffer (0.185 M Tris-HCI, 30% glycerol, 6% SDS, pH 6.8) at a ratio of 2:1. 20 mg of the protein solution was electrophoresed on a 10% polyacrylamide gel containing 0.5 mg/ml gelatin. After the electrophoresis, the resulting gel was washed with a washing buffer (2.5% Triton-X) for removing SDS contained in the gel. This procedure was repeated three times. Further, the gel was washed with a gel rinse buffer (50 mN Tris-HCl, 0.1 M NaCl, pH 7.4) for 20 minutes. Subsequently, an enzyme reaction was conducted at 37°C for 20 hours using a gel incubation buffer (50 mM Tris-HCI, 10 mM CaCl₂, 0.02% NaN₃). After the reaction, the gel was stained with a staining solution (0.5% CBB-G250, 45% methanol, 10% acetic acid), and further decolored with a decoloring solution (5% methanol, 7.5% acetic acid). The stained image was analyzed with Las-100 (FUJI FILM). The results are shown in an upper panel of Fig. 3.

Consequently, the MMP-9 activity was markedly suppressed in the tumor of the nude mouse inoculated with MEF gene stable overexpressing cell strain.

Further, 20 mg of the above supernatant was electrophoresed on a 10% acrylamide gel, followed by performed western blotting analysis in a usual manner using anti-MMP-9 antibody. The results are shown in a lower panel of Fig. 3.

Consequently, the MMP-9 expression was markedly inhibited in the tumor of the nude mouse inoculated with MEF gene stable overexpressing cell strain.

### Example 5

### (Influence on angiogenesis in an implanted tumor tissue)

### 1. Production of tumor sections

Two months after the inoculation of A549 and MEF gene stable overexpressing cell strain, tumor nodes formed subcutaneously in the nude mice were recovered, and freeze-stored using an OCT compound. The stored tissue was formed into sections with a thickness of 10 µm using a cryostat (LEICA CM 1100). Each section was attached to a poly-L-lysine-coated slide glass, which was dipped in a 4% paraformaldehyde aqueous solution, and the reaction was conducted for 15 minutes to immobilize the tissue, followed by dipped in PBS (-) for 5 minutes for washing. This procedure was repeated three times to produce a tissue specimen.

### 2. Hematoxylin-eosin staining

The tissue specimen was dipped in a Mayer hematoxylin-eosin solution for 3 minutes, and a color was brought out with distilled water. Further, the specimen was dipped in a 0.5% eosin for 1 minute, and then washed with distilled water. Subsequently, dehydration, removal of alcohol and inclusion were performed, and the specimen was microscopically observed. The photographs indicating the results are shown in Fig. 4. Consequently, angiogenesis was markedly inhibited in the tumor of the nude mouse inoculated with MEF gene stable overexpressing cell strain.

### Example 6 (Induction of apoptosis)

Apoptosis was detected in the above described tissue specimen using an apoptosis detection kit (Apoptosis in situ Detection kit wako (wako)). First, 100 µl of a TdT reaction solution diluted to 100 times was added to the tissue specimen, and the reaction was conducted in a wet box at 37°C for 15 minutes. Thereafter, the specimen was dipped in PBS (-) for 5 minutes for washing, and this procedure was repeated three times. Further, for inactivating an intrinsic peroxidase of the specimen, the specimen was reacted with a 3% H₂O₂ aqueous solution for 5 minutes. Then, the specimen was dipped in PBS (-) for 5 minutes for washing, and this procedure was repeated three times. Further, a POD-conjugated antibody was diluted to 100 times with PBS (-), 100 µl thereof was added dropwise to the specimen, and the reaction was conducted in a wet box at 37°C for 15 minutes. The specimen was then dipped in PBS (-) for 5 minutes for washing, and this procedure was repeated three times. Next, 100 µl of a DAB solution was added to a slide, and the reaction was conducted at room temperature for 5 minutes. Subsequently, the specimen was subjected to comparative staining with a methyl green acetic acid solution. Thereafter, the specimen was subjected to post dehydration, removal of alcohol and inclusion, and was microscopically observed. Photographs indicating the results are shown in Fig. 5.

Consequently, induction of apoptosis was markedly observed in the tumor of the nude mouse inoculated with MEF gene stable overexpressing cell strain.

### Example 7

### (Influence on a function of proliferating rabbit knee synovial membrane fibroblasts)

Synovial membrane fibroblasts prepared by outgrowth according to the Werb and Burleigh (1974) method from a knee joint synovial membrane extracted from a Japanese white rabbit (body weight approximately 3 kg) were used. The fibroblasts were proliferated in a CO₂ incubator adjusted to 37°C in a humid condition of 5% CO₂ and 95% air using MEM (Sigma) containing 10% inactive fetal calf serum, 25 mM HEPES, 100 U/ml penicillin G and 100 µg/ml streptomycin hydrochloride (hereinafter referred to as 10% FCS/MEM) as a culture solution. Subculture was performed in a usual manner using a 0.25% tripsin/0.02% EDTA solution. Synovial membrane fibroblasts at passage 3 to 7 were used for subculture on a 96-well plate at a cell density of 2,000 cells/0.1 ml/well, and incubated in a CO₂ incubator adjusted to 37°C in 10% FCS/MEM under a humid condition of 5% CO₂ and 95% air. After 24 hours of the incubation, MEF gene was transfected using a transfectam. After 24 hours from the transfection, human recombinant interleukin-1 (5 ng/ml) was added. Then, the function of inhibiting proliferation of synovial membrane fibroblasts after from 24 to 72 hours was examined by the MTT method. The results are shown in the above "Table 1".

Consequently, the remarkable function of suppressing proliferation of synovial membrane fibroblasts was observed in the fibroblasts with MEF transfected.

### Example 8 (RT-PCR method)

Isogene (Nippon Gene) was used in extraction of all RNAs from culture cells. For comparison of various gene expressions of culture cells, RT-PCR was conducted. RNA PCR kit (AMV) ver. 2.1 (TaKaRa) was used for RT-PCR. The methods by which the gene expression identified in the invention are outlined below.

### 1. MEF

A reverse transcription reaction (at 42°C for 60 minutes, at 99°C for 5 minutes, at 5°C for 5 minutes) was performed using 1 µg of all RNAs extracted from A549 and other cells as templates and oligo dT primers. Thereafter, PCR (at 94°C for 2 minutes - 1 cycle; at 94°C for 30 seconds, at 58°C for 30 seconds, at 72°C for 30 seconds - 40 cycles) was performed using an upstream primer (5'-GGAAGACCCCTCTGTGTTCCCAGCTG-3,) and a downstream primer (5,-CAGTCTTCTTGGCTCTTTCCTCTCTGG-3').

### 2. IL-8

A reverse transcription reaction (at 42°C for 60 minutes, at 99°C for 5 minutes, at 5°C for 5 minutes) was performed using 1 µg of all RNAs extracted from A549 and MEF gene overexpressing cell strain as templates and oligo dT primers. Thereafter, PCR (at 94°C for 2 minutes - 1 cycle; at 94°C for 30 seconds, at 58°C for 30 seconds, at 72°C for 30 seconds - 40 cycles) was performed using an upstream primer (5'-ATGACTTCCAAGCTGGCCGTGCT-3') and a downstream primer (5'-TCTCAGLCCTCTTCAAAAACTTCTC-3').

### 3. SE-3

A reverse transcription reaction (at 42°C for 60 minutes, at 99°C for 5 minutes, at 5°C for 5 minutes) was performed using 1 µg of all RNAs extracted from A549 and MEF gene overexpressing cell strain as templates and oligo dT primers. Thereafter, PCR (at 94°C for 2 minutes - 1 cycle; at 94°C for 30 seconds, at 55°C for 30 seconds, at 72°C for 30 seconds - 30 cycles) was performed using an upstream primer (5'-ACAGACAGCTACTCCACGTGCAATG-3') and a downstream primer (5'-CTCGTCTTTCCAGGTGTTCATGATGG-3').

### Example 9 (F-actin staining)

To cells incubated on a glass-bottom dish was added 4% paraformaldehyde, and the mixture was allowed to stand at room temperature for 10 minutes to immobilize the cells. Subsequently, the cells were permealized with 0.5% Triton X-PBS, and rhaodamine phalloidin (Molecular Probes) diluted to 50 times with PBS was added thereto. The reaction was conducted at room temperature for 30 minutes.

### Example 10 (Gelatin-zymography)

### 1. Preparation of a serum-free culture supernatant (conditioned media)

In the culture solution of the cells incubated to a subconfluent condition according to the foregoing cell incubation method, the incubation was replaced with serum-free incubation. After 48 hours of the incubation, the culture solution was recovered, and concentrated.

### 2. Preparation of a tumor lysate

An isolated tumor piece was placed into an 1.5-ml Eppendorf tube, and cut into small sections with scissors. Each section was suspended in a dissolution buffer (20 ml Tris-HCl, 0.1 M NaCl, 0.5% Triton X-100, pH 7.4), and the suspension was allowed to stand in ice for 1 hour. Subsequently, a supernatant was recovered by centrifugation of 2000 x g to give a tumor lysate.

### 3. Gelatin-zymography

Each of the samples prepared in 1. and 2. above and SDS sample buffer (0.185 M Tris-HCl, 30% glycerin, 6% SDS, pH 6.8) were mixed at a ratio of 2:1 to form a sample for zymography. This sample was electrophoresed on a 12.5% polyacrylamide gel containing 0.5 mg/ml gelatin. The gel after the electrophoresis was shaken in a gel washing solution (2.5% Triton X-100) at room temperature for 20 minutes to remove SDS. After this procedure was repeated three times, the gel was shaken three times in a gel rinse buffer (50 mM Tris-HCl, 0.1 M NaCl, pH 7.4) at room temperature for 5 minutes to remove Triton X-100. Thereafter, while the gel was shaken in a gel incubation buffer (50 mM Tris-HCl, 10 mM CaCl₂ or 10 mM EDTA, 0.02% NaN₃, pH 7.4) at 37°C for 20 hours, the enzyme reaction was conducted. After the reaction, the gel was stained with a gel staining solution (0.25% w/v CBB-G250, 45% methanol, 10% acetic acid), and then decolored with a gel decoloring solution (5% methanol, 7.5% acetic acid).

### Example 11 (Western blotting)

The sample used in the gelatin-zymography was employed as a sample. The sample was electrophoresed on a 12.5% polyacrylamide gel, and then transcribed on a PVDF membrane (250 mA, 1.5 hours). The transcribed PVDF membrane was shaken in 0.05% Tween 20-PGS (PBS-T) containing 5% skim milk at room temperature for 1 hour for masking. Subsequently, the membrane was shaken at room temperature for 1 hour using sheep anti-MMP-9 IgG (Santa Cruz) diluted to 1,000 times with PBS-T to conduct a primary antibody reaction. After the primary antibody reaction, the membrane was shaken using an anti-sheep antibody labeled with mouse HRP (Jackson ImmunoResearch Laboratories, Inc.) diluted to 10,000 times at room temperature for 1 hour to conduct a secondary antibody reaction. After the second antibody reaction, the antibody reaction was detected with an ECL reagent (Amersham).

### Example 12

### (Measurement of an ability to invade cells into a matrigel)

200 µl of each cell suspension prepared to a concentration of 1.0 x 10⁶ cells/ml was added to an upper layer of a matrigel invasion chamber (BD Biocoat Matrigel) having 8-µm pores, and 700 µl of a serum-free culture solution was added thereto. After 24 hours, the cells invaded in the lower layer of the membrane filter were stained with hematoxylin, and the number of the cells was then counted under a microscope.

### Example 13 (Measurement of a cell proliferation rate)

Using WST Kit (Dojindo), a cell proliferation rate was measured according to its protocol. The outline is described below. 1.0 x 10⁴ cells were inoculated in a 24-well plate, and incubated for 4 days. To the cells on day 0 and day 4 was added a WST reagent diluted to 20 times with the culture solution, and the reaction was conducted for 2 hours. Subsequently, an absorbance of 405 nm was measured on the respective cells. The absorbance on day 4 was divided by the absorbance on day 0 to give a cell proliferation rate.

### Example 14 (Method for forming colonies in a soft agar)

To a 6-well plate was added 2 ml of a 0.5% agar-DMEM solution, and it was allowed to stand at room temperature until solidified to form a lower layer. Subsequently, 2 ml of a mixed solution obtained by mixing the cells with a 0.33% agar-DMEM solution to a concentration of 2.5 x 10⁴ cells/ml was added to a lower layer, and solidified at room temperature to form an upper layer. Further, 1 ml of the culture solution was added to the resulting upper layer, and incubated in 5% CO₂ at 37°C for 2 weeks. Then, colonies formed were observed under a microscope.

### Example 15 (Immunological tissue staining)

### 1. PECAM-1

To the tissue specimen formed was added 4% paraformaldehyde, and the resulting specimen was allowed to stand at room temperature for 10 minutes for immobilization. Then, rat anti-PECAM-1 IgG (Parmingen) diluted to 100 times with PBS-T in a usual manner was added thereto, and a primary antibody reaction was conducted at 4°C for 16 hours. After the primary antibody reaction, a secondary antibody reaction was conducted at room temperature for 1 hour using anti-rat IgG (Vector Laboratories) labeled with rabbit biotin and diluted to 100 times with PBS-T. Comparative staining was performed with a 3% methyl green acetic acid solution. Incidentally, with respect to quantitative determination of angiogenesis, four fields with 200 x magnification were selected at random, and the number of vessels having a size of 50 µm or more or a size of 50 µm or less was counted to find an average value.

### 2. MMP-9

To the tissue specimen produced was added 5% paraformaldehyde, and the resulting specimen was allowed to stand at room temperature for 10 minutes for immobilization. Then, sheep anti-MMP-9 IgG (Santa Cruz) diluted to 150 times with PBS-T in a usual manner was added thereto, and a primary antibody reaction was conducted at 4°C for 16 hours. After the primary antibody reaction, a secondary antibody reaction was conducted at room temperature for 1 hour using anti-rat IgG (Vector Laboratories) labeled with rabbit biotin and diluted to 100 times with PBS-T. Comparative staining was performed with a 3% methyl green acetic acid solution. Example 16 (Measurement of a floating ability of HUVEC)

200 µm of a cell suspension containing HUVECs at a concentration of 2.5 x 10⁵ cells/ml was added to an upper layer of a matrigel invasion chamber (BD Biocoat Matrigel) having 8-µm pores. Subsequently, 700 µl of a serum-free culture supernatant produced according to 1. of Example 10 was added to a lower layer. After 24 hours, HUVECs invaded into the lower layer of the membrane filter were stained with hematoxylin, and the number of cells was then counted under a microscope.

### Example 17 (Preparation of a reporter gene)

### 1. Preparation of MMP-9 reporter gene

A luciferase gene was used as a reporter gene for measuring a transcription activity of MMP-9 in cells. First PCR (at 94°C for 30 seconds, at 58°C for 30 seconds, at 72°C for 30 seconds - 30 cycles) was performed using human genomic DNA as a template and 5'-MMP-9pro (primer) and 3'-MMP-9pro (primer). Further, second PCR (at 94°C for 30 seconds, at 58°C for 30 seconds, at 72°C for 30 seconds - 30 cycles) was performed using the resulting PCR product as a template, 5'-MMP-9pro2 (primer 2) and 3'-MMP-9pro2 (primer 2). The resulting PCR product was inserted into PCR 2.1 (Invitrogen), then treated with restriction enzymes Kpn I and Xho I, and inserted into Kpn I and Xho I sites of pGL2 basic vector.

### 2. Preparation of IL-8 reporter gene

A luciferase gene was used as a reporter gene for measuring a transcription activity of IL-8 in cells. First PCR (at 94°C for 30 seconds, at 54°C for 30 seconds, at 72°C for 30 seconds - 30 cycles) was performed using human genomic DNA as a template, 5'-IL-8pro (primer) and 3'-IL-8pro (primer). The resulting PCR product was inserted into PCR 2.1 (Invitrogen), then treated with restriction enzymes Hind III and Xho I, and inserted into Hind III and Xho I sites of pGL 2 basic vector.

### Example 18

### (Construction of Ets transcription factor protein expression gene)

pCB6 was used as a protein expression vector for examining influence of human MMP-9 and IL-8 on a transcription activity. Subcloning of Ets transcription factor in pCB6 was conducted in the following manner, and a base sequence of DNA was identified by a cycle sequence method.

### 1. pCB6/MEF

cDNA was produced from mRNA of cancer cell strain NCI-H292 derived from an airway epithelial cell. PCR (at 94°C for 1 minute, at 64°C for 1 minute, at 65°C for 1.5 minutes - 40 cycles; at 72°C for 20 minutes - 1 cycle) was performed using this cDNA as a template, 5'-MEF (BamH I) and 3'-MEF (EcoR I). The resulting PCR product was cloned in pCR2.1 vector (pCR 2.1/MEF) with Original TA Cloning Kit). This clone was treated with restriction enzymes Hind III and Xba I, and inserted into Hind III and Xba I sites of pCB6 vector to obtain pCB6/MEF vector.

### 2. pCB6/ESE-1

pC1/ESE-1 (supplied from Dr. T. A. Libermann) was treated with restriction enzymes Hind III and Xba I, and inserted into Hind III and Xba I sites of pCB6 to obtain pCB6/ESE-1 vector.

### 3. pCB6/EIf-1

PCR (at 94°C for 1 minute, at 60°C for 1 minute, at 72°C for 1.5 minutes - 40 cycles; at 72°C for 20 minutes - 1 cycle) was performed using pUC118/E1f-1 cloned from human heart-derived cDNA library as a template, 5'-EIf-1 (Kpn I) and 3'-EIf-1 (Xba I). The resulting PCR product was treated with restriction enzymes Kpn I and Xba I, and inserted into Kpn I and Xba I sites of pCB6 vector to obtain pCB6/EIf-1 vector.

### 4. pCB6/Ets-1

PCR (at 94°C for 1 minute, at 60°C for 1 minute, at 72°C for 1.5 minutes - 40 cycles; at 72°C for 20 minutes - 1 cycle) was performed using pBluescrIpt/Ets-1 (supplied from Dr. D. K. Watson) as a template, 5'-ets1 (Bgl II) and 3'-ets1 (Hind III). The resulting PCR product was treated with restriction enzymes Bgl II and Hind III, and inserted into Bgl II and Hind III sites of pCB6 vector to obtain pCB6/Ets-1 vector.

### 5. pCB6/Ets-2

PCR (at 94°C for 1 minute, at 60°C for 1 minute, at 72°C for 1.5 minutes - 40 cycles; at 72°C for 20 minutes - 1 cycle) was performed using pBluescript/Ets-2 (supplied from Dr. D. K. Watson), 5'-ets2 (Bgl II) and 3'-ets2 (Hind III). The resulting PCR product was treated with restriction enzymes Bgl III and Hind III, and inserted into Bgl II and Hind III sites of pCB6 vector to obtain pCB6/Ets-2 vector.

### 6. pCB6/PEA3

pGEM/PEA3 (supplied from Dr. J. A. Hassel) was treated with restriction enzyme Xba I, and inserted into Xba I site of pCB vector to obtain pCB6/PEA3 vector.

### 7. pCB6/antisense MEF

pCB6/MEF plasmid was treated with restriction enzymes EcoR I and BamH I. The resulting DNA fragment of approximately 2 kbp was purified, and then subjected again to a ligation reaction. The resulting plasmid was treated with restriction enzymes to identify an insert direction, and desired plasmid DNA with MEF cDNA introduced in an antisense direction was obtained.

### 8. pCB6/antisense ESE-1

pC1/ESE-1 (supplied from Dr. T. A. Libermann) was treated with restriction enzymes Hind III and Kpn I, and inserted into Hind III and Kpn I sites of pCB6 vector to obtain pCB6/antisense ESE-1 vector.

### Example 19

### (Transfection of a reporter gene into cells and measurement of a promoter activity)

### (1) Transfection of a reporter gene and a protein expression gene into cells

Transfection of DNA into cells was conducted with Transfectam and LT1 according to protocols thereof. The methods thereof are outlined below.

### 1. Transfectam

500 ng of reporter plasmid DNA, 0.5 µg-5.0 µg of protein expression plasmid DNA and 2 µl-10 µl of a Transfectam solution were mixed, and a total amount was adjusted to 150 µl with DMEM. This mixed solution was reacted at room temperature for 10 minutes, and then added to sub-confluent cells on a 24-well plate. For collecting a transfection efficiency between the cells, pRL-CMV vector (10 ng/well) was co-transfected. After incubation at 37°C for 2 hours, 0.5 ml of 10% FBS+DMEM was freshly added, and introduced.

### 2. LT1

50 µl of Opti-MEM and 6 µl of LT1 were mixed, and allowed to stand at room temperature for 10 minutes. This mixed solution was added to a DNA solution containing 500 ng of reporter plasmid DNA and 0.5 µg-5.0 µg of protein expression plasmid DNA, and the reaction was conducted for 10 minutes. Subsequently, the reaction solution was added to sub-confluent cells on a 24-well plate. For collecting a transfection efficiency between the cells, pRL-CMV vector (10 ng/well) was co-transfected. Incidentally, an amount of a plasmid DNA sample was obtained from an absorbance of UV (260 nM), and the sample having a purity of 85% or more was used.

### (2) Measurement of a promoter activity

A promoter activity in the cells transfected with the reporter gene was measured using a dual-luciferase reporter assay system (Promega). A medium was removed from the cells incubated for 48 hours, and the residue was washed twice with cold PBS (-). 100 µl of the cell solution was added, and the mixture was moderately shaken at room temperature for 15 minutes. Subsequently, the cell solution was recovered, and 20 µl of this solution and 100 µl of a luminous solution were mixed at room temperature. A luciferase activity was measured with a luminometer (Lumat LB9507, eg & g berthtold).

### Industrial Applicability

The invention discloses that the ETS transcription factor or the gene encoding the same, preferably the ETS transcription factor MEF protein or the gene encoding the same is effective as a cell proliferation inhibitor or an MMP production inhibitor, more specifically as an MMP-9 production inhibitor. The cell proliferation inhibitor or the MMP production inhibitor of the invention, more specifically the MMP-9 production inhibitor or the IL-8 production inhibitor is useful as anti-tumor therapeutic methods and anti-tumor therapeutic agents of malignant tumors, namely, small-cell pulmonary cancer, non-small-cell pulmonary cancer, breast cancer, rectum cancer, digestive organ cancer, large bowel cancer, cervical cancer, bladder cancer, ovarian cancer, prostatic cancer, multiple myeloma, chronic myeloid leukemia and malignant lymphoma, and antirheumatic agents.

## Claims

1. A cell proliferation inhibitor comprising an ETS transcription factor or a gene encoding the same, or a substance regulating a function of the ETS transcription factor or the gene encoding the same.

2. The cell proliferation inhibitor as claimed in claim 1, wherein the ETS transcription factor or the gene encoding the same is an ETS transcription factor MEF protein or a gene encoding the same.

3. The expression regulator as claimed in claim 1 or 2, wherein the substance regulating the function of the ETS transcription factor or the gene encoding the same is a substance regulating the MEF protein or the gene encoding the same.

4. The cell proliferation inhibitor as claimed in claim 2 or 3, wherein the ETS transcription factor MEF protein is a protein comprising an amino acid sequence described in SEQ ID No. 1 of SEQUENCE LISTING, or the amino acid sequence in which one or more amino acids are substituted, deleted or added.

5. The cell proliferation inhibitor as claimed in claim 2 or 3, wherein the ETS transcription factor MEF protein or the gene encoding the same is a gene having a nucleotide sequence described in SEQ ID No. 2 of SEQUENCE LISTING or the nucleotide sequence in which one or more nucleotides are substituted, deleted or added, or a nucleotide sequence capable of hybridizing to these nucleotide sequences under stringent conditions.

6. The cell proliferation inhibitor as claimed in any one of claims 1 to 5, wherein the cell is an epithelial cell.

7. The cell proliferation inhibitor as claimed in any one of claims 1 to 6, wherein the cell is a cancer cell.

8. The cell proliferation inhibitor as claimed in any one of claims 1 to 6, wherein the cell is a synovial membrane cell.

9. The cell proliferation inhibitor as claimed in any one of claims 1 to 7, which is an antitumor agent.

10. The cell proliferation inhibitor as claimed in any one of claims 1 to 6 and 8, which is an antirheumatic agent.

11. An MMP production inhibitor or an IL-8 production inhibitor comprising an ETS transcription factor MEF protein or a gene encoding the same, or a substance regulating a function of the MEF protein or the gene encoding the same.

12. The MMP production inhibitor as claimed in claim 11, wherein MMP is MMP-9.

13. A method for screening a substance capable of being a cell proliferation inhibitor, in which a cell having transfected therein a gene encoding an ETS transcription factor is used, and a test substance is added to the cell or around the cell to measure an expression amount of the gene encoding the ETS transcription factor in the cell.

14. The method as claimed in claim 13, wherein the gene encoding the ETS transcription factor is a gene encoding an MEF protein.

15. The method as claimed in claim 13 or 14, comprising a method for screening a substance capable of being an active ingredient of an antitumor agent or an antirheumatic agent.

16. An antitumor agent comprising a substance regulating a function of an ETS transcription factor or a gene encoding the same.

17. The antitumor agent as claimed in claim 16, wherein the substance regulating the function of the ETS transcription factor or the gene encoding the same is a substance regulating a function of an MEF protein or a gene encoding the same.

18. The antitumor agent as claimed in claim 16 or 17, wherein the substance regulating the function of the ETS transcription factor or the gene encoding the same is the substance obtained by the method as claimed in any of claims 13 to 15.

19. An antirheumatic agent comprising a substance regulating a function of an ETS transcription factor or a gene encoding the same.

20. The antirheumatic agent as claimed in claim 19, wherein the substance regulating the function of the ETS transcription factor or the gene encoding the same is a substance regulating a function of an MEF protein or a gene encoding the same.

21. The antirheumatic agent as claimed in claim 19 or 20; wherein the substance regulating the function of the ETS transcription factor or the gene encoding the same is the substance obtained by the method as claimed in any of claims 13 to 15.


SEQUENCE LISTING

<110> Hisamitsu Pharmaceutical Company

<110> Kai, Hirofumi

<120> CELL PROLIFERATION INHIBITORS COMPRISING ETS TRANSCRIPTION FACTOR
      OR GENE ENCODING THE SAME

<130> JA339318

<150> JP2001-034834

<151> 2001-02-13

<160> 2

<210> 1

<211> 663

<212> PRT

<213> Human

<400> 1

<210> 2
<211> 4190
<213> DNA
<214> Human

<400> 2
   gaattccctt tcgccggcgc cgagttcctg gcgccgctcg cccggcccgg cttccgaggg 60
   gagaggacgg gctggcgggg ctggggaccc gcgtctcggc ccccggagcg gggaccacgg 120
   agacagaccc cggcccggcg accgagctgg gcccgtgagc cactcggcct caggtcgctc 180
   ctgtggttgg tccagcccag aatgcagcct tgagcctggc ttaggccacc acctactcca 240
   gctctctcca ccccctattt tactgcagct cagggggtag gctctaggct ccaaagtacc 300
   tgggtattgt cccttcatca agaaagcccc acagctctgg agggctctga taatcccgtt 360
   gtcagctctc tgaaaagaca gcatggctat taccctacag cccagtgacc tgatctttga 420
   gttcgcaagc aacgggatgg atgatgatat ccaccagctg gaagacccct ctgtgttccc 480
   agctgtgatc gtggagcagg taccctaccc tgatttactg catctgtact cgggactgga 540
   gttggacgac gttcacaatg gcatcataac agacgggacc ttgtgcatga cccaggatca 600
   gatcctggaa ggcagttttt tgctgacaga tgacaatgag gccacctcgc acaccatgtc 660
   aaccgcggaa gtcttactca atatggagtc tcccagcgat atcctggatg agaagcagat 720
   cttcagtacc tccgaaatgc ttccagactc ggaccctgca ccagctgtca ctctgcccaa 780
   ctacctgttt cctgcctctg agcccgatgc cctgaacagg gcgggtgaca ctagtgacca 840
   ggaggggcat tctctggagg agaaggcctc cagagaggaa agtgccaaga agactgggaa 900
   atcaaagaag agaatccgga agaccaaggg caaccgaagt acctcacctg tcactgaccc 960
   cagcatcccc attaggaaga aatcaaagga tggcaaaggc agcaccatct atctgtggga 1020
   gttcctcctg gctcttctgc aagacagaaa cacctgtccc aagtacatca agtggaccca 1080
   gcgagagaaa ggcatcttca aactggtgga ctccaaagct gtgtccaagc tgtgggggaa 1140
   gcagaaaaac aagcctgaca tgaactatga gacaatgggg cgggcactaa gatactacta 1200
   ccaaagaggc atactggcca aagtggaagg gcagaggctg gtgtaccagt ttaaggagat 1260
   gcccaaggac ctggtggtca ttgaagatga ggatgagagc agcgaagcca cagcagcccc 1320
   acctcaggcc tccacggcct ctgtggcctc tgccagtacc acccggcgaa ccagctccag 1380
   ggtctcatcc agatctgccc cccagggcaa gggcagctct tcttgggaga agccaaaaat 1440
   tcagcatgtc ggtctccagc catctgcgag tctggaattg ggaccgtcgc tagacgagga 1500
   gatccccact acctccacca tgctcgtctc tccagcagag ggccaggtca agctcaccaa 1560
   agctgtgagt gcatcttcag tgcccagcaa catccaccta ggagtggccc ccgtggggtc 1620
   gggctcggcc ctgaccctgc agacgatccc actgaccacg gtgctgacca atgggcctcc 1680
   tgccagtact actgctccca ctcagctcgt tctccagagt gttccagcgg cctctacttt 1740
   caaggacacc ttcactttgc aggcctcttt ccccctgaac gccagtttcc aagacagcca 1800
   ggtggcagcc ccaggggctc cactgattct cagtggcctc ccccaacttc tggctggggc 1860
   caaccgtccg accaacccgg cgccacccac ggtcacaggg gctggaccag cagggcccag 1920
   ctctcagccc cctgggactg tcattgctgc cttcatcagg acttctggca ctacagcagc 1980
   ccctagggtc aaggaggggc cactgaggtc ctcctcctat gttcagggta tggtgacggg 2040
   ggcccccatg gaggggctgc tggttcctga agagaccctg agggagctcc tgagagatca 2100
   ggctcatctt cagccacttc caacccaggt ggtttccagg ggttcccaca atccgagcct 2160
   tctgggcaac cagactttgt ctcctcccag ccgccccact gttgggctga ccccagtggc 2220
   tgaacttgag ctctcctcag gctcagggtc cctgctgatg gctgagccta gtgtgaccac 2280
   atctgggagc cttctgacaa gatcccccac cccagcccct ttctccccat tcaaccctac 2340
   ttccctcatt aagatggagc cccatgacat ataagcaaag gggtcagggc aagtgtgacc 2400
   caccaggcaa aattgagcag cattttcata gggaccgact tcagtagcac acctgcccct 2460
   gcatttcagt gggatgtcaa tacacttgac cccaagtccc ccggccctgc ctggtgtcac 2520
   tgtggccaaa cagtgcccag cttaagcatc cctggcatca gactatggcc ttcaagagca 2580
   ctagggcata tgcttttggc agcataacgg gctgacttgg tgatggaggg aaaaagcctt 2640
   gagccaggca gaagtttgtg gccagggttt gtgcagcagc tttgtgagaa gagcccttct 2700
   acctggctct atctcactgg ctgcattccc tacacaggga atttactacc ctatatgtga 2760
   atatcccctg tatgtacttg tgtgtacttg ttggtctgta tcttagtttc tttggggagg 2820
   acagggctgt agctgtgagg tcttgtctcc aagggtgtgt gtatgtctcc gtggatcagc 2880
   cacagggata gggattttgt ttttaaggga aagcattctc taattccctt tgttcatgcc 2940
   gagattcagt tgctctgaga ctatggggta caagtttgat cctccgaatc tggagatgtt 3000
   gtagagctgg aacgagtgca gagtaggaac gctttgatgc gcatgcacat tggggaagat 3060
   gcgctcctca gggacacaaa ggccgagtgg ggtaaaacca cgaagggagg gaagggaagt 3120
   cagctctggg agcagccctc actggctgga ccaaggtact cttcctggag tttgccgtgt 3180
   tagcaaccac agtcaccttg cagtcaggct ggaatcttgg gccaccccac agtgctttgc 3240
   tgtaggattt agacggggat gaagtgccct ccagcctcag agctagccac aaagccccca 3300
   gagctgaatt cattgagtat ttgtgcctag ggcttgggct gtttgtgtga taccggcccc 3360
   ccgacagaca ataggctgtg atgacacccc agtctacttc cccgatcctg ggctccctct 3420
   tgattagtag gtgacatttt ccactgtcag gcatcactgg ggctagtccg gcagcgacct 3480
   agatggggtc cacccccatt cctgctcaag catgggcacc taccacatgg tttctgctgc 3540
   tcagcctgac tgcaactcac ctcgaaggcg gaccagcctg cctctgtgat gactgcagaa 3600
   gacctccttg ggtgtaccaa tgcccctcat ctcccacttt cacacctaac cctgactcct 3660
   tcaccaagaa gacgggagtc ggcagccagg agttcccgtg gcacctctct ctcttcgtgg 3720
   ctccctgctt cccccttccc tctttccgag gaagggtcaa cctattctct ctcaaaacca 3780
   acccctaggc caattgcctg gatctcctcc cctctccctt ctttaaacga gcttgcctcc 3840
   ctcctgccaa gtttgagggc aaggctaaga aatgtcagcc acggaaacaa ctctaatatc 3900
   tggtgacttt gggtaatgtg aatcagtgcc tgaggacctt tgctgtgtcc ttggtacaga 3960
   accatccact tgacctaact acctcccctg gccgcgctct cgctcttctc ttctttgtta 4020
   agccaacaac tatcaccctc tcctactctt ctttctccct gccccctgga gggcactgtg 4080
   tttggttgtg caaatgtatt tactatgcgt gtttccagca gttggcatta aagtgccttt 4140
   ttctaataaa atcagtttat tatgaccaaa aaaaaaaaaa aaaggaattc 4190